(19) Europäisches Patentamt
European Patent Office
Office européen des brevets

(11) **EP 3 720 428 B1**

(12) # EUROPEAN PATENT SPECIFICATION

(45) Date of publication and mention of the grant of the patent:
**11.01.2023 Bulletin 2023/02**

(21) Application number: **18811023.3**

(22) Date of filing: **29.11.2018**

(51) International Patent Classification (IPC):
***A61K 31/185*** *(2006.01)* ***A61P 27/02*** *(2006.01)*

(52) Cooperative Patent Classification (CPC):
**A61K 31/185; A61P 27/02**

(86) International application number:
**PCT/EP2018/082999**

(87) International publication number:
**WO 2019/110416 (13.06.2019 Gazette 2019/24)**

(54) **OPHTHALMIC TOPICAL COMPOSITION COMPRISNG DOBESILIC ACID FOR TREATING DISEASES OF THE POSTERIOR SEGMENT OF THE EYE**

OPHTHALMISCHE TOPISCHE ZUSAMMENSETZUNG ENTHALTEND DOBESILSÄURE ZUR BEHANDLUNG VON ERKRANKUNGEN DES HINTEREN AUGENSEGMENTS

COMPOSITION TOPIQUE OPHTALMIQUE COMPRENENT DE L’ACIDE DOBESILIQUE POUR LE TRAITEMENT DES MALADIES DU SEGMENT POSTÉRIEUR DE L’ IL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **04.12.2017 EP 17382832**

(43) Date of publication of application:
**14.10.2020 Bulletin 2020/42**

(73) Proprietor: **Laboratorios SALVAT, S.A.**
**08950 Esplugues de Llobregat, Barcelona (ES)**

(72) Inventors:
- **CAPDEVILA NORTES, Xavier**
  **08950 Esplugues De Llobregat (ES)**
- **SANAGUSTÍN AQUILUÉ, Javier**
  **08950 Esplugues De Llobregat (ES)**
- **DELGADO GAÑÁN, Maria Isabel**
  **08950 Esplugues De Llobregat (ES)**

(74) Representative: **ZBM Patents - Zea, Barlocci & Markvardsen**
**Rambla Catalunya, 123**
**08008 Barcelona (ES)**

(56) References cited:
**EP-A1- 2 777 699 EP-A1- 2 875 811**

- **J Nirmal ET AL: "Evaluation of stability and in vivo trans-corneal penetration of 1% topical calcium dobesilate formulation", Indian Journal of Pharmacology 41st Annual Conference of Indian-Pharmacological-Society; New Delhi, INDIA; December 18 -20, 2008, 1 October 2008 (2008-10-01), XP055474373, Retrieved from the Internet: URL:https://www.ncbi.nlm.nih.gov/pmc/articles/PMC3086146/ [retrieved on 2018-05-11]**
- **VELPANDIAN T ET AL: "EVALUATION OF CALCIUM DOBESILATE FOR ITS ANTI-CATARACT POTENTIAL IN EXPERIMENTAL ANIMAL MODELS", METHODS AND FINDINGS IN EXPERIMENTAL AND CLINICAL PHARMACOLOGY,, vol. 32, no. 3, 31 March 2010 (2010-03-31) , pages 171-179, XP009505296, ISSN: 0379-0355, DOI: 10.1358/MF.2010.32.3.1423888**
- **P. CUEVAS ET AL: "Topical dobesilate eye drops for ophthalmic primary pterygium", BMJ CASE REPORTS, vol. 2012, no. mar08 1, 8 March 2012 (2012-03-08), XP055474235, UK ISSN: 1757-790X, DOI: 10.1136/bcr.12.2011.5449**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 720 428 B1

- **PEDRO CUEVAS ET AL: "Improvement in the signs and symptoms of dry eye disease with dobesilate eye drops", MILITARY MEDICAL RESEARCH, vol. 2, no. 1, 1 December 2015 (2015-12-01), XP055474245, DOI: 10.1186/s40779-015-0068-8**
- **CRISTINA HERN?NDEZ ET AL: "Topical administration of GLP-1 receptor agonists prevents retinal neurodegeneration in experimental diabetes", DIABETES, 17 September 2015 (2015-09-17), page db150443, XP055422205, US ISSN: 0012-1797, DOI: 10.2337/db15-0443**
- **RAFAEL SIMÓ: "Mechanisms of retinal neuroprotection of calcium dobesilate: therapeutic implications", NEURAL REGENERATION RESEARCH, vol. 12, no. 10, 1 October 2017 (2017-10-01), pages 1620-1622, XP055474254, cited in the application**
- **RANTA VELI-PEKKA ET AL: "Barrier analysis of periocular drug delivery to the posterior segment", JOURNAL OF CONTROLLED RELEASE, vol. 148, no. 1, 7 September 2010 (2010-09-07), pages 42-48, XP029172598, ISSN: 0168-3659, DOI: 10.1016/J.JCONREL.2010.08.028**


Remarks:

The complete document including Reference Table(s) and the Sequence Listing(s) can be downloaded from the EPO website

## Description

This application claims the benefit of European Patent Application 17382832.8 filed the 4th of December of 2017.

## Technical Field

The invention relates to the field of medical approaches for treating diseases of the eye posterior chamber, including retinopathies as well as pathologies of the optic nerve. The invention also relates to ophthalmic compositions.

## Background Art

Eye is a complex organ with different specialized cells and tissues, which are regulated to maintain optimal visual function. This function is achieved with the presence of tight barriers in the anterior and posterior segments of the eye, which play a key role by selective control of inward and/or outward traverse of fluids and solutes. Local and systemic diseases may affect the different regions of the eye, but the anatomy of the eye and complex physiology of the retina and optic nerve makes challenging the development of efficacious drugs.

Eye is broadly divided into two compartments called the anterior segment (front of the eye) and posterior segment (back of the eye). The anterior segment of the eye refers to the cornea, conjunctiva, anterior sclera (part of sclera that transitions anteriorly to become cornea at the limbus) iris, ciliary body, aqueous humour and the lens. The posterior segment of the eye (also named posterior eye segment) includes the anterior hyaloid membrane and the structures behind this membrane, posterior sclera (part of sclera that transitions posteriorly into optic nerve dural sheat), the vitreous body (including vitreous humour and membrane), retina, macula, choroid and optic nerve. Posterior segment represents the two-thirds of the eye. The leading causes of vision impairment and irreversible blindness are diseases related with the posterior segment of the eye.

The least invasive route for ocular drug administration are topical formulations (e.g. eye drops). Topical local administration is the main modality treatment for anterior segment disease. However, ocular barriers for avoiding pathogens to access also hinder drug delivery even to the anterior segment. In addition, blinking and tear film turnover, designed to wash away foreign material and maintain a smooth clear anterior surface, also limits residence time of a drug. Furthermore, access to posterior segment is hindered by a closely packed corneal epithelium and stroma with varying lipophilicity (see Awwad et al., "Principles of Pharmacology in the Eye", British Journal of Pharmacology-2017, vol no. 174 Issue 23, pp.:4205-4223). Further, the direction of aqueous flow in the eye (ciliary body to anterior chamber angle) is against direction of drug delivery via a topical route.

Notwithstanding, if a drug can finally reach the posterior segment of the eye, it may be removed from the vitreous cavity through diffusion into the anterior chamber, or by the blood-retinal barrier. All this makes difficult any eye treatment of a disease of the posterior segment by topically administration onto eye surface, and the only local modalities in everyday use for treating diseases of the posterior segment of the eye are intravitreal injection (IVT) or periocular injections. IVT injections avoid all barriers and results in the greatest bioavailability. However, IVT injections imply the small but significant risk of blinding complication, the risk of retinal detachment and of infective endophtalmitis. Besides, these injections represent a highly aggressive mode of treatment for initial stages of the diseases.

On the other hand, systemic formulations for treating diseases of posterior segment of the eye have to overcome blood retinal barrier (BRB), that controls fluid and molecular movement between the ocular vascular beds, and prevents leakage of macromolecules (e.g. albumin) and harmful molecules in the retina. Due to the outer and inner layer of BRB, influx of any drug into the retina and vitreous is highly limited. This means that many formulations require of high doses of drug with the accompanying adverse side-effects.

Main posterior segment ophthalmic disorders include such conditions as pathological neovascularization and ectopic proliferation, atrophy and nerve cell death, inflammation and infection, and detachment. Diseases and conditions commonly associated with these symptoms include macular degeneration, diabetic retinopathy, retinopathy of prematurity, retinitis pigmentosa, macular edema, glaucoma, posterior uveitis, endophthalmitis, ocular insult and ocular manifestation of systemic disease such as viral infection, arthritis and rosacea.

Calcium dobesilate monohydrate (CDO) orally administered is known as the treatment of diabetic retinopathy (DR) and of venous insufficiency. Doxium® (Laboratorios Dr. Esteve) is the commercial name of the hard capsules of calcium dobesilate monohydrate that are administered for non-proliferative diabetic retinopathy.

CDO is the monohydrate of calcium salt of dobesilic acid, also termed 2,5-dihydroxybenzenesulfonic acid monohydrate. Calcium dobesilate has the CAS number 20123-80-2. CDO (CAS number, 117552-78-0) has the following formula (I):

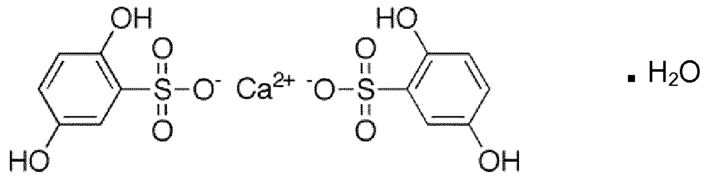

(I)

It is widely known that CDO is a compound that can be easily oxidized to calcium 3,6-dioxocyclohexa-1,4-diene-1-sulfonate and thus it is highly unstable. This physico-chemical property of CDO supposes a drawback for the topical administration since it quickly oxidises. In addition CDO is an extremely hydrophilic molecule, what is an outstanding barrier for the ocular drug absorption. It is well known that the corneal epithelium is lipoidal in nature and poses a significant resistance for permeation of topically administered hydrophilic drugs, furthermore the conjunctival epithelial tight junctions can further retard passive movement of hydrophilic molecules (Gaudana et al, "Ocular drug delivery", American Association of Pharmaceutical Sci. 2010 Sep;12(3):348-360. doi: 10.1208/s12248-010-9183-3. Epub 2010 May 1). The corneal and conjunctival epitheliums are physiological barriers that avoid the penetration of high hydrophilic molecules as dobesilic acid and its salts. Furthermore the drug penetration into the posterior ocular tissues is mainly governed by the blood retinal barrier (BRB) which is selectively permeable to highly hydrophobic molecules, being another limiting step to the highly soluble molecules like CDO. Due to the limited bioavailability of polar molecules some of the strategies to enhance transcleral retinal delivery ocular drug absorption have been focussed in the development hydrophobic prodrugs (Vadlapudi et al, "Ocular Drug Delivery", Chapter 10, pp.:2019-263).

A CDO similar compound, in particular, the salt of dobesilic acid diethylamine 2,5-dihydroxybenzenesulfonate (Etamsylate, Dicynone®, Sanofi-Aventis) has been disclosed in patent application EP2875811A1 as active ingredient of IVT injections for the treatment of submacular and subvitreous haemorrhages. IVT injections of Dicynone® have been also disclosed in patent EP2777699B1 for treating age-related macular degeneration. Both documents propose using the ocular surface as an alternative to the invasive IVT injections, although data are not illustrated. This is mainly because due to the above-mentioned barriers imposed by anterior segment of the eye, drugs are unlikely to reach the posterior segment of the eye. Yet more unlikely if the drug is highly polar or hydrophilic, and it has to pass through the variable lipophilicity of the different tissues of this anterior segment. Also due to their solubility, soluble drugs are quickly washed by tears. If in addition it is taken into account that the drug is quickly oxidised, local IVT injection seems the best option.

For all these reasons, besides the systemic administration of CDO (Doxium®) for treating DR, other dobesilate compounds are proposed nowadays for these IVT injections in the treatment of diseases of posterior segment of the eye, but there are no compositions for topical eye administration of these compounds. (See for example Cuevas et al., "Single Intravitreal Injection of Etamsylate for the Treatment of Geographic Atrophy Associated with Submacular Hemorrhage", MOJ Clin Med Case - 2017, vol. no.7(1): 00186; Cuevas et al., "Intravitreal Dobesilate Injection for Macular Oedema Secondary to Branch Retinal Vein Occlusion", MOJ Clin Med Case - 2016, vol. no.4(1): 00078).

Salts of dobesilic acid are used in the treatment of diseases of the posterior segment of the eye, whether administered orally or injected, mainly due to its vasoprotection effect and angioprotective effect. CDO is effective for at least the following mechanisms of action: (i) reducing retinal albumin leakage and capillary permeability, which protects the blood-retinal barrier (BRB); (ii) inhibiting platelet aggregation and blood viscosity; (iii) up-regulating endothelium-dependent relaxation because of an increase in nitric oxide synthesis; (iv) inhibiting apoptosis of vascular endothelial cells in blood vessels; (v) antioxidant and antiradical activity; (vi) protecting against reactive oxygen species; (vii) prevents the upregulation of ICAM-1 that regulates the inflammation and the vascular endothelial growth factor, (viii) anti-inflammatory action or (ix) neuroprotection. It is as well described that etamsylate is a hemostatic (reduction of bleeding time) that reduces intraocular pressure in rabbits and inhibits the biosynthesis of prostaglandins.

These mechanisms of action stand on the nature of dobesilic acid (salts or esters of said acid or salts), which is a compound with high antioxidant properties, mainly because it oxidises first, as indicated above. It is also due to the fact that it oxidises quickly, that the salt of dobesilic acid diethylamine 2,5-dihydroxybenzenesulfonate (Etamsylate, Dicynone®, Sanofi-Aventis) is injected intravitreal in the previously referred trials. Any other topical ocular application is not advisable according to prior art, mainly because its high polarity, low expected absorption and limited residence time once locally administered.

A skilled person would in any case propose dobesilic acid as oral drug (systemic), according to the teaching of Simó et al., "Mechanisms of retinal neuroprotection of calcium dobesilate: therapeutic implications" Neural Regen Res -2017, vol. no. 12(10), pp.:1620-1622. Nonetheless, Simó et al. do also state that systemic treatments addressed to block main pathways involved in the diabetic retinopathy pathology, among which dobesilic acid is proposed, can barely reach the retina and optic nerve at pharmacological concentrations.

Furthermore there is a scientific clear acceptance that the topical eye drop administration is useful only for the

treatment of anterior segment diseases of the eye and the treatment of posterior eye segment is an important therapeutic target with unmet medical needs (del Amo et al., "Current and future ophthalmic drug delivery systems. A shift to the posterior segment", Drug Discovery Today- 2008, vol. no. 13(3/4), pp.: 135-143).

Therefore, there is still a need of non invasive alternative route of administration for topical ocular compositions for the prophylactic and therapeutic treatment of posterior segment ophthalmic disorders that are capable of delivering a therapeutically effective amount of an active ingredient to the posterior segment. Also needed are methods for prophylactic and therapeutic treatments of posterior segment ophthalmic disorders.

**Summary of Invention**

Inventors realised that dobesilic pharmaceutically acceptable salts, in particular CDO and etamsylate salt, can be topically administered onto eye surface, being able to pass quickly all the barriers of anterior segment of the eye, and reach the retina and the optical nerve (fundus of the eye). Thus, when topically administered, they pass through cornea, conjunctiva and sclera, but also overcome the barrier of iris and lens. This finding is surprising and unexpected, since highly hydrophilic molecules like CDO show poor transcorneal permeability and barely reach the posterior chamber of the eye. Furthermore the high water solubility may involve a fast dissolution or dilution of active ingredient in the tear fluids and a fast rinse and clearance, reducing the absorption and bioavailability of the drug after ocular topical administration. Outstandingly, despite its antioxidant activity and high reactivity, dobesilic acid salts are not degraded in the diffusion pathway from the ocular surface to the inner ocular tissues like retina and/or optic nerve. In addition, CDO remains in several structures of the posterior segment of the eye for a period of time long enough to perform their effects.

Therefore, although they can be unstable due to oxidation processes, and although being highly polar and soluble in polar solvents, dobesilic acid and/or its salts, such as CDO and etamsylate, as well as esters of said acid or salts, reach posterior segment of the eye and remain there to act as vasoprotective and neuroprotective agents due to its action as antiangiogenic agents, among other properties.

These findings are highly advantageous since reveal that the administration of CDO onto the ocular surface allows an effective route of administration to treat disorders of the posterior eye segment that are an important therapeutic target with unmet medical needs.

In addition, as it is shown in the examples, the CDO topically applied has a high bioavailability in posterior eye segment, higher than this of a single oral dose. This is quite surprising, because it is widely accepted that due to the several barriers to be overcome to reach posterior segment, the bioavailability of a drug in vitreous humour and retina after ophthalmic topical administration is very low and consequently "topical eye drops do not deliver drug effectively to the retina and choroid" (Ranta et al., "Barrier analysis of periocular drug delivery to the posterior segment", Journal of Controlled Release-2010, vol. no. 148, pp.: 42-48). Or, as indicated by Awwad et al. "Principles of Pharmacology in the Eye", British Journal of Pharmacology-2017, vol. no. 174 Issue 23, pp.: 4205-4223, the drugs applied in a conventional topical way are diluted by a factor between 250,000 and 1,000,000 by the time the vitreous is reached. Thus if a particular drug is able to reach vitreous humor once topically administered, the amounts are pharmacologically inefficient.

Also surprising is the fact that low concentrations of the dobesilic acid salts (e.g. CDO or etamsylate at 1% w/v) topically administered onto the surface of the eye (cornea, conjunctiva, anterior sclera, iris and ciliar body) give amounts of the drug in retina and optical nerve up to six hours after topic administration. The assayed concentrations (1 and 10% w/v) are lower that the proposed for intravitreal injections of etamsylate disclosed in EP2875811A1 and EP2777699B1 (12.5 % diethylamine 2,5-dihydroxybenzenesulfonate).

Finally, the data also demonstrated that CDO (100 mg/ml) administered topically has a good tolerance.

Thus, a first aspect of the invention is a dobesilic acid and/or a pharmaceutically acceptable salt thereof, or ester of any of them, for use in the treatment and/or prevention of a disease of the posterior segment of the eye, wherein the treatment comprises administration of a topical dose onto eye front surface of the dobesilic acid and/or a pharmaceutically acceptable salt or ester thereof from 0.01 mg/day to 400 mg/day.

The effect of dobesilic acid and/or of any of their salts, or esters of them, stands on its role as vasoprotector, antioxidant, antiapoptotic, anti-inflammatory, neuroprotector, and antiangiogenic among other properties. Thus, they are for use in the particular treatment and/or prevention of any disease of the posterior segment of the eye related to the referred activities.

To the best of inventor’s knowledge this is the first time dobesilic acid or a derivative thereof (salt or ester of both, the acid or the salt) is proposed for treatment or for prevention of diseases of the posterior segment of the eye by topical administration onto eye front surface.

This new therapeutically approach solves the problems of side-effects associated to systemic administration, or the problems of the aggressive IVT injections currently applied.

In addition, the therapeutically effective dose range from 0.01 mg/day to 400 mg/day, supposes non-toxic doses well below the doses that would be considered effective. Thus, as above indicated, the bioavailability of a drug in vitreous humour and retina after ophthalmic topical administration is very low and, although effective amounts between different

routes of administrarion are not comparable, in case one would desire delivering to posterior segment the same amount/day of a drug that is injected intravitreally, any topical composition should contain at least in a 250,000-fold amount/day in relation with the composition for intravitreal injection. This would make topical opthalmic pharmaceutical compositons highly concentrated and dangerous (i.e toxic) for other eye structures (e.g. those structures of anterior segment of the eye).

This first aspect can also be formulated as the use of dobesilic acid and/or of a pharmaceutically acceptable salt, or ester of any of the salt or the acid, in particular the salts calcium dobesilate or etamsylate (diethylamine 2,5-dihydroxybenzenesulfonate), for the manufacture of a medicament for the treatment and/or prevention of a disease of the posterior segment of the eye, wherein the treatment comprises administration of a topical dose onto eye surface of the dobesilic acid and/or a pharmaceutically acceptable salt, or ester of any of them, from 0.01 mg/day to 400 mg/day. The present invention also relates to a method for the treatment and/or prevention of a disease of the posterior segment of the eye comprising topically administering onto eye surface (cornea, conjunctiva, anterior sclera, iris and ciliary body) a therapeutically effective amount of dobesilic acid and/or a pharmaceutically acceptable salt, or ester thereof, together with pharmaceutically topical acceptable excipients and/or carriers, in a subject in need thereof, including a human, and in which the treatment comprises administration of a topical dose of the dobesilic acid and/or a pharmaceutically acceptable salt or ester thereof from 0.01 mg/day to 400 mg/day.

The topical treatment and/or prevention, according to this invention, means that is administered onto the eye surface (i.e. in the cornea, anterior sclera, and the other tissues of the anterior segments such as the iris and ciliary body or conjunctival fornix). It is due to the fact that the dobesilic acid and/or salts, or esters of both, can reach the retina and optic nerve and other tissues of the posterior segment of the eye when applied topically to eyes. This applies to any of the embodiments and combination of embodiments disclosed in the present invention, as well as to any composition comprising said dobesilic acid and/or pharmaceutically acceptable salt, or ester thereof.

A second aspect of the invention is a pharmaceutical topical eye composition for use in the treatment and/or prevention of a disease of the posterior segment of the eye, which comprises a therapeutically effective amount of dobesilic acid and/or of a pharmaceutically acceptable salt, or ester of said acid or said salt, together with one or more pharmaceutically acceptable topical excipients and/or carriers; wherein the treatment comprises administration of a topical dose of the dobesilic acid and/or a pharmaceutically acceptable salt, or ester thereof, from 0.01 mg/day to 400 mg/day. Particular topical eye pharmaceutical compositions comprising dobesilic acid and/or pharmaceutically acceptable salts thereof (such as CDO or etamsylate) have been developed in such a way that, once administered onto the surface of the eye, they allow said acid or pharmaceutically acceptable salts, or esters thereof, to reach the retina and optic nerve in effective amounts. Other structures of the posterior segment of the eye are also reached (e.g. choroids, posterior sclera, humour vitreous, etc.).

Thus, an embodiment relates to a pharmaceutical topical eye composition, comprising a therapeutically effective amount of dobesilic acid and/or a pharmaceutically acceptable salt, or an ester of the acid or the salt, together with one or more pharmaceutically acceptable topical eye excipients and/or carriers, the composition formulated for the release of dobesilic acid and/or a pharmaceutically acceptable salt, or an ester of any of the acid or the salt, from 2 to 30 days.

These pharmaceutical topical eye compositions of sustained release of the active principle imply the advantages of reducing the number of administrations to get effective amounts in target tissues and for a desired period of time.

## Brief description of the drawings

Figure 1 is a graphic with bars showing concentrations of oxidized glutathione (GSSG) in homogenized retinas of non-diabetic rats (Ctrl), diabetic rats induced by streptozotocin (STZ), and in diabetic rats induced by streptozotocin treated with CDO 10 mg/ml; topical instillation of 10 μl/eye.

Figure 2 is a graphic with bars showing relative mRNA levels of the inflammatory cytokine TNF-α in retinas of non-diabetic rats (Ctrl), diabetic rats induced by streptozotocin (STZ), and diabetic rats induced by streptozotocin treated with CDO 10 mg/ml; topical instillation of 10 μl/eye. *Fold incr.* vs control means increase in relation to control.

Figure 3. Photographies of the HET-CAM ocular irritation assay. Evaluation of lysis, haemorrhage or coagulation processes in the egg chorioallantoic membrane (CAM) after the application of NaCl 0.9% (negative control), NaOH 0.1% (positive control), CDO (100 mg/ml), etamsylate (100 mg/ml) and potassium dobesilate (100 mg/ml).

## Detailed description of the invention

All terms as used herein in this application, unless otherwise stated, shall be understood in their ordinary

meaning as known in the art. Other more specific definitions for certain terms as used in the present application are as set forth below and are intended to apply uniformly through-out the specification and claims unless an otherwise expressly set out definition provides a broader definition.

For "retinal and/or choroid pathologies", it is to be understood any disease or disorder affecting retina and/or choroid. Under this definition there are included diseases and disorders of different outcome and symptomatology. Examples include, as a non-limitative list, retinal vasculopathies, maculopathies, hereditary eye fundus dystrophies, idiopatic chorioretinopathies, central serous retinopathies, generalized choroidal dystrophies, and combinations thereof. In the particular case of choroid pathologies, they refer to diseases affecting the choroid, which is the vascular layer of the eye, containing connective tissues, and lying between the retina and the sclera. Particular diseases include the inflammatory one chorioretinitis, or choroiditis if only choroid is inflamed; posterior uveitis, which is the inflammation of the uvea, the pigmented layer that lies between the inner retina and the outer fibrous layer composed of the sclera and cornea; and general choroidal dystrophy, including choroideremia and choroidal atrophy.

Particular examples of retinal "vasculopathies" (pathologies in which directly or indirectly blood vessels irrigating retina are involved) include the widely known diabetic retinopathy, diabetic papillopathy, non-diabetic retinopathy (including as main disorder the macular edema caused by uveitis or retinal vascular disease), ocular ischemic syndrome, hypertensive retinopathy, thalassemia retinopathy, Coats' syndrome, eales' syndrome, radiation retinopathy, solar retinopathy, purtscher retinopathy, polypoidal choroidal vasculopathy (PCV), retinal macroaneurysm, retinal microaneurysm, leukemic retinopathy, retinal ischemia (in particular due to central retinal vein occlusion, branch retinal vein occlusion, branch retinal artery occlusion, and retinopathy of prematurity), and chronic retina disorders (such as acute retinal necrosis syndrome and Birdshot retinopathy). Among them "Diabetic retinopathy (DR)" is the leading cause of blindness among working age adults. It is observed in up to ninety percent of patients with insulin dependent diabetes mellitus of long-term duration (more than 10 years). Initially, the high blood glucose levels common to persons with diabetes mellitus cause an increase in glycosated proteins and growth factor levels in the eyes. This condition known as the "pre-diabetic retinopathy stage" can lead to retinopathy if not prophylactically treated. Non-proliferative or early-stage diabetic retinopathy, also known as "background diabetic retinopathy," is characterized by thickening of the basement membrane, loss of retinal pericytes, microvascular abnormalities, intraretinal microaneurysms, retinal hemorrhages (known as "dot blot" or "cotton wool" spots), retinal edema, capillary closure, and soft and hard exudates. Late-stage or proliferative diabetic retinopathy is characterized by neovascularization and fibrovascular growth, i.e., scarring involving glial and fibrous elements, from the retina or optic nerve over the inner surface of the retina or into the vitreous cavity. In the sense of the invention, DR includes Non-proliferative or early-stage diabetic retinopathy and Late-stage or proliferative diabetic retinopathy. In addition, the disorders known as diabetic macular edema or diabetic maculopathy, and fundus diabetic retinopathy are also encompassed in the meaning of diabetic retinopathy.

Particular examples of "maculopathies", which are pathological condition of the macula, the area at the centre of the retina that is associated with highly sensitive, accurate vision include, among others, aged-related macular degeneration (ARMD), hemorrhagic ARMD, retinal angiomatous proliferation, polipoidal choroidal vasculopathy, malattia leventinese, full thickness macular hole, macular epiretinal membrane, macular telangiectasias, cellophane maculopathy or macular pucker, myopia maculopathy, exudative maculopathy after venous thrombosis of the retina, acute macular neuroretinopathy, macular cystoid macular edema, retinal angioid streaks, choroidal folds, and hypotony maculopathy. "Age-related macular degeneration (AMD or ARMD)" also known as "macular degeneration", is a medical condition which may result in blurred or no vision in the center of the visual field. Early on there are often no symptoms. Over time, however, some people experience a gradual worsening of vision that may affect one or both eyes. While it does not result in complete blindness, loss of central vision can make it hard to recognize faces, drive, read, or perform other activities of daily life. There is an atrophic form (also known as dry AMD, in which early signs include a build-up of material called drusen between the RPE and Bruch's membrane which impairs diffusion of oxygen from the choroidal circulation.

Advanced disease is characterized by RPE and photoreceptor cell death often in localized regions of the peripheral retina (geographic atrophy), as well as alterations in the Bruch's membrane that prevents the proliferation and reattachment of RPE cells. On the other hand there is wet form (wet-AMD) characterized by loss of central vision, usually in both eyes, due to damage to the retinal pigment epithelial (RPE) cells and invasion of the avascular outer retina by immature blood vessels from the choroid capillaries. The invasion involves a breach of the RPE cell layer which constitutes the blood-retinal barrier. When invasion involves the macula, patients have serious visual impairment. The natural course of neovascularization in ARMD is the development of a disciform scar over the macula and irreversible blindness. When in present invention AMD is referred it encompasses any of the forms dry- and wet-AMD.

"Hereditary eye fundus distrophies" relate to hereditary diseases including retinitis pigmentosa; atypical retinitis pigmentosa, including but not limited to Usher's syndrome, retinitis punctata albicans, Leber's congenital amaurosis; dystrophy of the cones; rod dystrophy; Bietti crystalline corneoretinal dystrophy; juvenile macular dystrophy; all types of macular dystrophy; Stargardt's disease or Fundus flavimaculatus; and Usher's Syndrome.

For "vitreous humour pathologies" is to be understood as any disorder or disease of the vitreous structure, including the vitreous body (gel filling space between the lens and the retina of the eyeball of humans and other vertebrates)

and the vitreous membrane. These disorders include eye floaters, myodesopsia, which are blood, cells or other byproducts of inflammation get into the vitreous; asteroid hyalosis, sub-macular and vitreous haemorrhages; and vitreous detachment, which takes place when vitreous pulls away from the retina; as well as hereditary vitreoretinopathies, including but not limited to retinoschisis, Stickler's syndrome or Wagner's syndrome.

"Optic nerve pathologies" relate to pathologies affecting optic nerve for many causes but leading to dysfunction of the mechanism allowing transmission of visual information from the retina to the brain. Among these pathologies there are included optic atrophy; the optic neuritis (inflammation of the optic nerve due to demyelinating, infectious or non-infectious ethiology); neuroretinitis; and ischemic optic neuropathy (sudden loss of blood supply and nutrients to the optic nerve head); hereditary optic neuropathy (such as Leber's hereditary optic neuropathy); toxic amblyopia or nutritional optic neuropathy, ocular hypertension, primary glaucoma (including primary open-angle glaucoma and primary angle-closure glaucoma); secondary Glaucoma, including but not limited to Neovascular glaucoma, inflammatory glaucoma or traumatic glaucoma; iridocorneal endothelial syndrome associated to glaucoma, optic nerve head drusen or optic disc drusen; and papilledema; and trauma. There are included herewith lesions due to other eye diseases finally affecting the optic nerve, such as glaucoma (increase of eye pressure that impairs vision due to the pressure receiving also the nerve). "Glaucoma" is in turn a group of diseases characterized by a particular pattern of blindness involving damage to the optic nerve and visual field loss. Increased intra-ocular pressure is a risk factor and contributes to some but not all cases of optic nerve atrophy and retinal cell death in the glaucomatous eye. Even when elevated intraocular pressure (IOP) is not exhibited, glaucomatous cell death occurs in the retina. Optic nerve damage occurs when the separations between axon bundles in the optic nerve and the retinal nerve fiber layer degenerates.

"Posterior sclera pathology" relates to scleritis, which is the inflammation of the sclera in the region of the posterior eye segment. Posterior scleritis is defined as involvement of the sclera posterior to the insertion of the rectus muscles. Posterior scleritis, although rare, can manifest as serous retinal detachment, choroidal folds, or both. There is often loss of vision as well as pain upon eye movement.

In the sense of the present invention, a "vascular disease of the posterior segment of the eye" includes any disease or alteration of normal function of any of the tissues and/or structures constituting the posterior segment of the eye, said diseases or alterations being caused at least partially due to an impairment of the vascular system supplying blood to said eye structures. Therefore, this term includes diseases or disorders that standing from many and different aetiologies (genetic predisposition, trauma, other metabolic diseases such as diabetes, etc.) they have in common one or more of a dysfunction of blood vessels irrigating eye (such as the retinal albumin leakage and capillary permeability, platelet aggregation and increased blood viscosity, impaired endothelium-dependent relaxation, etc.); an impaired antioxidant and antiradical activity of cells; the presence of reactive oxygen species; inflammation and neurodamage.

The expression "therapeutically effective amount" as used herein, refers to the amount of a compound that, when administered, is sufficient to prevent development of, or alleviate to some extent, one or more of the symptoms of the disease which is addressed. The particular dose of compound administered according to this invention will of course be determined by the particular circumstances surrounding the case, including the compound administered, the route of administration, the particular condition being treated, and the similar considerations.

The term "pharmaceutically acceptable" as used herein pertains to compounds, materials, compositions, and/or dosage forms which are, within the scope of sound medical and veterinary judgment, suitable for use in contact with the tissues of a subject (e.g. human or any other animal) without significant toxicity, irritation, allergic response, or other problem or complication, commensurate with a reasonable benefit/risk ratio. Each carrier, excipient, etc., must also be "acceptable" in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio. Suitable carriers, excipients, etc. can be found in standard pharmaceutical texts, and include, as a way of example preservatives, pH adjusting agents, tonicity agents to adjust osmolality, chelating agents, penetration enhancers, bioadhesive polymers, stabilizers, viscosizing agents, and antioxidants.

The term "pharmaceutically acceptable excipients and/or carriers" or "pharmaceutically acceptable topical eye excipients and/or carriers" (used interchangeable) refers to that excipients or carriers suitable for use in the pharmaceutical technology for preparing compositions with medical topical eye use, that is, that can be applied onto eye surface without significant toxicity, irritation, allergic response, or other problem or complication, commensurate with a reasonable benefit/risk ratio.

The term "weight/volume percentage" refers to the weight in grams of a compound per 100 ml of composition, for instance, between the whole composition and dobesilic acid and/or a pharmaceutically acceptable salt, or of and ester thereof, in said composition. It is represented as % w/v along the description, or only as %.

The term "pH" is defined as the value given by a suitable, properly standardized, potentiometric sensor and measuring system. The measuring system has traditionally been referred to as the "pH meter". The pH of the compositions are measured by compendial traditional methods.

As above indicated, a first aspect of the invention relates to the dobesilic acid and/or a pharmaceutically acceptable salt, or ester of said acid or salt, for use in the treatment and/or prevention of a disease of the posterior segment of the eye, wherein the treatment comprises administration of a topical dose onto eye surface of the dobesilic acid and/or a pharmaceutically acceptable salt or ester thereof from 0.01 mg/day to 400 mg/day.

In a particular embodiment, the pharmaceutically acceptable salt of dobesilic acid is selected from an alkali metal salt of dobesilic acid, an alkaline earth metal salt of dobesilic acid, a salt of dobesilic acid with an amine of formula (II), and combinations thereof

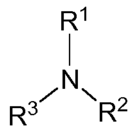 (II),

wherein $R^1$, $R^2$ and $R^3$ are independently selected from H, and /or -($C_1$-$C_8$)-alkyl radicals.

Throughout the description and claims, the term "-($C_1$-$C_8$)-alkyl radical", shall be construed as straight or branched. It includes, any of the radicals methyl, ethyl, n-propyl, iso-propyl, cyclopropyl, n-butyl, sec-butyl (or 1-methylpropyl), isobutyl (or 2-methylpropyl), tert-butyl (or 1,1-dimethylethyl), n-pentyl, tert-pentyl (or 2-methylbutan-2-yl), neopentyl (or 2,2-dimethylpropyl), isopentyl (or 3-methylbutyl), sec-pentyl (or pentan-2-yl), 3-pentyl (or pental-3-yl), n-hexyl, isohexyl (or 4-methylpentyl), tert-hexyl, sec-hexyl, n-heptyl, tert-heptyl, isoheptyl, sec-heptyl, n-octyl, tert-octyl, sec-octyl, and isooctyl.

In a particular embodiment, the compound used in the ocular treatment is a pharmaceutically acceptable salt of dobesilic acid selected from an alkali metal salt of dobesilic acid, an alkaline earth metal salt of dobesilic acid, and combinations thereof. More in particular is an alkaline earth metal salt of dobesilic acid. Even more in particular, it is the calcium salt of dobesilic acid, also named calcium dobesilate. More in particular is CDO (calcium dobesilate monohydrate). Thus, in this later particular embodiment the invention relates to calcium dobesilate or a hydrate thereof (such as the monohydrate CDO) for use in the treatment and/or prevention of a disease of the posterior segment of the eye. Other pharmaceutical acceptable salts of dobesilic acid include magnesium salt, potassium salt and sodium salt.

The salts of dobesilic acid may be in crystalline form either as free solvation compounds or as solvates (e.g. hydrates) and it is intended that both forms are within the scope of the present invention. Methods of solvation are generally known within the art. As above exposed particular solvates include hydrated salts, more in particular mono- and dihydrated sals of dobesilic acid, as well as esters of said salts.

In another particular embodiment of the first aspect of the invention, the pharmaceutically acceptable salt of dobesilic acid is a salt of dobesilic acid with an amine of formula (II), wherein $R^1$ and $R^2$ are equal and are ethyl radicals and $R^3$ is H. This salt of dobesilic acid is also known as etamsylate or as diethylamine 2,5-dihydroxybenzensulfonate.In another particular embodiment, etamsylate is an anhydrous form. In another particular embodiment, etamsylate is in hydrate form, more in particular selected from etamsylate monohydrate and etamsylate dihydrate.

"Esters of dobesilic acid or esters of dobesilic acid salts" refer to pharmaceutically acceptable esters formed from pharmaceutically acceptable non-toxic organic acids. There is no limitation regarding the ester, except that if used for therapeutic purposes, they must be pharmaceutically acceptable. Esters of dobesilic acid may be prepared from pharmaceutically acceptable non-toxic organic acids. Such acids include among others acetic, butyric, propionic, citric, fumaric, gluconic, glutamic, lactic, maleic, malic, mandelic, succinic and tartaric acid. Examples of ester groups include acetyl, ethanoyl, butyryl, propyionyl, acetoxymethyl, methoxymethyl, succinyl, and the like, as well as ester groups derived from the coupling of the hydroxyl group at any of carbon atoms at positions C-2 and C-5 of the 2,5-dihydroxybenzensulfonic acid compound of this invention with the above mentioned organic acids. In particular, an example of ester of dobesilic acid is the 2,5-diacetylbenzensulfonic acid and/or salts of 2,5-diacetylbenzensulfonic acid. Other particular examples of esters are pharmaceutically acceptable esters formed from the coupling of the hydroxyl group at any of carbon atoms at positions C-2 and C-5 of the 2,5-dihydroxybenzensulfonic acid with pharmaceutically acceptable non-toxic organic acids of general formula COOH-($C_1$-$C_3$)alkyl, wherein ($C_1$-$C_3$)alkyl is selected from the radicals methyl, ethyl, n-propyl, iso-propyl and cyclopropyl.

In a particular embodiment, optionally in combination with any embodiments or aspects above or below, the dobesilic acid and/or pharmaceutically acceptable salt, or ester thereof, is for topical use in the treatment of a disease of the posterior eye segment selected from retinal and/or choroid pathologies, vitreous humour pathologies, posterior sclera pathologies, optic nerve pathologies, and combinations thereof.

As above indicated, the use of dobesilic acid and/or of a pharmaceutically acceptable salt thereof, or esters of both, in pathologies of retina, vitreous humour, choroid and optic nerve, lies on the fact that these compounds are vasoprotective agents, antioxidant, antiapoptotic, anti-inflammatory, neuroprotector, hemostatic, and antiangiogenic among other properties. Thus, they are for use in disorders affecting these structures that have been injured or functionally impaired due to vascular problems (e.g. vasoconstriction, obstruction of vessels, loss of endothelium-dependent relaxation, high permeability of capillary vessels, etc.), or damages related to stress oxidative conditions, inflammatory processes or apoptosis in the above referred tissues.

In a particular embodiment of the first aspect of the invention, also in combination with any embodiments above or below, the disease of the posterior segment of the eye is a retinal and/or choroid pathology selected from a retinal vasculopathy, a maculopathy, an hereditary eye fundus dystrophy, an idiopatic chorioretinopathy, a central serous retinopathy, generalized choroidal dystrophy, and combinations thereof.

In yet a more particular embodiment, the retinal vasculopathy is selected from diabetic retinopathy, diabetic papillopathy, non-diabetic retinopathy, ocular ischemic syndrome, hypertensive retinopathy, thalassemia retinopathy, Coats' syndrome, Eales' syndrome, radiation retinopathy, solar retinopathy, Purtscher retinopathy, polypoidal choroidal vasculopathy (PCV), retinal macroaneurysm, retinal microaneurysm, leukemic retinopathy, retinal ischemia, chronic retina disorders, and combinations thereof. More in particular is for use in diabetic retinopathy, including proliferative diabetic retinopathy, non-proliferative diabetic retinopathy, both with any accompanying disorder, in particular diabetic macular edema and/or fundus diabetic retinopathy. Even more in particular it is for use in early stages of diabetic retinopathy, when neurodegeneration starts and produces functional abnormalities, such as the loss of both chromatic discrimination and contrast sensitivity even before of the detection of microvascular lesions under ophthalmologic examination.

In another particular embodiment of the first aspect of the invention, also in combination with any embodiments above or below, the retinal and/or choroid pathology is a maculopathy selected from aged-related macular degeneration (ARMD), including dry and wet-ARMD; hemorrhagic ARMD; retinal angiomatous proliferation; polipoidal choroidal vasculopathy; malattia leventinese; full thickness macular hole; macular epiretinal membrane; macular telangiectasias; cellophane maculopathy or macular pucker; myopia maculopathy; exudative maculopathy after venous thrombosis of the retina; acute macular neuroretinopathy; macular cystoid macular edema; retinal angioid streaks; choroidal folds, hypotony maculopathy and combinations thereof.

In another particular embodiment of the first aspect of the invention, also in combination with any embodiments above or below, the retinal and/or choroid pathology is an hereditary eye fundus distrophy selected from the group consisting of retinitis pigmentosa; atypical retinitis pigmentosa, including but not limited to Usher's syndrome, retinitis punctata albicans, Leber's congenital amaurosis; dystrophy of the cones; rod dystrophy; Bietti crystalline corneoretinal dystrophy; juvenile macular dystrophy; all types of macular dystrophy; Stargardt's disease or Fundus flavimaculatus; Usher's Syndrome and combinatios thereof.

In another particular embodiment of the first aspect of the invention, optionally in combination with any embodiments or aspects above or below, the dobesilic acid and/or pharmaceutically acceptable salt thereof, or an ester of them, is for use in the treatment, by topical administration onto the eye surface of these compounds, of a vitreous humour pathology selected from the group consisting of sub-macular and vitreous haemorrhages, asteroid hyalosis, vitreous detachment, eye floaters or myodesopsia, hereditary vitreoretinopathies, including but not limited to retinoschisis, Stickler's syndrome or Wagner's Syndrome, and combinations of all these.

Another particular embodiment of the first aspect of the invention is the dobesilic acid and/or a pharmaceutically acceptable salt, or ester of any of them, for use in a disease of the posterior segment of the eye, which is an optic nerve pathology selected from the group consisting of optic atrophy; optic neuritis, including demyelinating, infectious or non-infectious; neuroretinitis; ischemic neuropathy; hereditary optic neuropathy (ej. Leber's hereditary optic neuropathy); toxic amblyopia or nutritional optic neuropathy; ocular hypertension; primary glaucoma; secondary glaucoma, including but not limited to neovascular glaucoma, inflammatory glaucoma or traumatic glaucoma; Iridocorneal endothelial syndrome associated to glaucoma; optic nerve head drusen or optic disc drusen; papilledema (or papilloedema), and combinations thereof.

In a more particular embodiment, the disease of the posterior eye segment is selected from retinal and/or choroid pathologies, optic nerve pathologies, sclera and combinations thereof, since in these tissues of the posterior segment of the eye high amounts of dobesilic acid and/or its salts are detected once administered topically onto the eye surface.

As will be depicted in the examples below, CDO and etamsylate topically administered onto the surface of the eyes of rabbits or rats were able to reach posterior sclera, vitreous humour, choroid, retina and optic nerve. Thus, a particular embodiment of the invention relates to calcium dobesilate or etamsylate for use in the treatment and/or prevention of a disease of the posterior segment of the eye selected from choroid pathologies, vitreous humour, retinal pathologies, optic nerve pathologies, and combinations thereof, wherein the treatment comprises administration of a topical dose onto eye surface of the dobesilic acid and/or a pharmaceutically acceptable salt or ester thereof from 0.01 mg/day to 400 mg/day.

CDO, as well as another pharmaceutically acceptable salt or ester of dobesilic acid, surprisingly permeates into the posterior segment of the eye and remains there for exercising its action for a long period of time. As indicated in previous paragraphs, calcium dobesilate or another pharmaceutically acceptable salt or ester of dobesilic acid promotes the prophylaxis and/or treatment of all these diseases due to its role as vasoprotective agents, in particular by reducing

retinal albumin leakage and capillary permeability, which protects the blood-retinal barrier (BRB); by up-regulating endothelium-dependent relaxation because of an increase in nitric oxide synthesis; inhibiting apoptosis of vascular endothelial cells in blood vessels; by antioxidant and antiradical activity; protecting against reactive oxygen species (ROS); antiinflamatory activity, hemostatic or preventing the upregulation of ICAM-1 and vascular endothelial growth factor. In addition, all these biochemical effects are transferred to neuroprotection of neurons in retina and optic nerve.

In another particular embodiment of the first aspect of the invention, the dobesilic acid and/or a pharmaceutically acceptable salt, or ester of any of them, is for use in a disease of the posterior segment of the eye in a mammal, and more in particular in humans.

In yet another particular embodiment of the first aspect, the topical dose of the dobesilic acid and/or a pharmaceutically acceptable salt, or of an ester of any of them, is from 0.01 mg/day to 300 mg/day, even more in particular from 0.1 mg/day to 300 mg/day. More in particular, from 0.1 mg/day to 100 mg/day. In another particular embodiment, it is from 0.01 mg/day to 50 mg/day. In yet another particular embodiment, it is from 0.01 mg/day to 25 mg/day. In another embodiment, it is from 0.01 mg/day to 20 mg/day. More in particular from 0.01 mg/day to 18 mg/day, even more in particular from 0.1 mg/day to 18 mg/day. In another particular embodiment, it is from 0.01 mg/day to 13 mg/day, more in particular from 0.1 mg/day to 13 mg/day.

Dobesilic acid or any pharmaceutically acceptable salt, as well as any ester of any of the acid or salts, can be the active ingredient of different types of pharmaceutical topical eye compositions. Thus, the invention also relates, in a second aspect, to pharmaceutical topical eye compositions for use in the treatment and/or prevention of a disease of the posterior segment of the eye, which comprises a therapeutically effective amount of dobesilic acid and/or of a pharmaceutically acceptable salt, or an ester of any of the acid or the salt, together with one or more pharmaceutically acceptable topical excipients and/or carriers; and wherein the treatment comprises administration of a topical dose of the dobesilic acid and/or a pharmaceutically acceptable salt, or of an ester of any of them, from 0.01 mg/day to 400 mg/day.

In a particular embodiment of the pharmaceutical topical eye composition for use as indicated above, said composition comprises dobesilic acid and/or a pharmaceutically acceptable salt of dobesilic acid, or an ester of said acid or salt, in a percentage by weight/volume from 0.02 to 20% w/v. More in particular, the percentage by weight/volume is from 0.05% to 15% w/v. More in particular it is selected from 0.05%, 0.1%, 0.5%, 1%, 2%, 3%, 4%, 5%, 6%, 7%, 8%, 9%, 10%, 11%, 12%, 13%, 14%, and 15% w/v. More in particular it is from 0.05% to 10% w/v. In another particular embodiment, the percentage of dobesilic acid and/or of a pharmaceutically acceptable salt of dobesilic acid, or of an ester of said acid or salt, is from 11% to 20% w/v.

In a particular embodiment, the composition for use as indicated above comprises a pharmaceutically acceptable salt of dobesilic acid, more in particular is an alkali metal or alkaline earth metal salt of dobesilic acid. Even more in particular, it comprises calcium dobesilate. More in particular is CDO (calcium dobesilate monohydrate). Other pharmaceutical acceptable salts of dobesilic acid include magnesium salt, potassium salt and sodium salt.

In another particular embodiment of the second aspect of the invention, the pharmaceutically acceptable salt of dobesilic acid is a salt of dobesilic acid with an amine of formula (II), wherein $R^1$,and $R^2$ are equal and are ethyl radicals and and $R^3$ is H. This salt of dobesilic acid is also known as etamsylate or as diethylamine 2,5-dihydroxybenzensulfonate. Hydrated salts of etamsylate are also encompassed.

In another particular embodiment, the pharmaceutical topical eye composition for use as indicated above is formulated for giving a dose from 0.01 mg/day to 400 mg/day, by topical administration onto eye surface. The capability of delivering the active principle could be modulated by excipients and/or carriers accompanying it. The excipients must be selected from those that do not degrade the active ingredient in order to prepare the topically onto eye administrable pharmaceutical composition for the intended use of the invention. Suitable carriers, excipients, etc. can be found in standard pharmaceutical texts, and include, as a way of example preservatives, pH adjusting agents, tonicity agents to adjust osmolality, chelating agents, penetration enhancers, tensioactives, bioadhesive polymers, stabilizers, viscosizing agents, and antioxidants.

In yet another particular embodiment, the pharmaceutical topical eye composition for use as disclosed above comprises an antioxidant compound.

The antioxidant compound is selected from the group consisting of butylated hydroxyanisole, butylated hydroxytoluene, tert-butylhydroquinone, tertiary butylhydroquinone, dilauryl thiopropionate, ethoxyquin, propyl gallate, 2,4,5-trihydroxybutyrophenone, thiodipropionic acid, alpha-tocopherol, ascorbic acid, sodium ascorbate, potassium sulphite, potassium hydrogen sulphite potassium metabisulfite, potassium bisulfite, potassium sorbate, sodium benzoate, sodium iodide, sodium metabisulfite, sodium bisulfite, sodium hydrogen bisulfite, calcium sulphite, calcium hydrogen sulphite, sodium succinate hexahydrate, sodium sulfite, sodium tartrate, sodium thiosulfate, sorbic acid, trisodium citrate dihydrate, and mixtures thereof.

Topical ocular dosage forms of the pharmaceutical composition for use according to the invention are those appropriated for allowing the active ingredient to be stable and to be applied topically onto the surface of eye (cornea, conjunctiva, anterior sclera, iris or ciliary body). In a particular embodiment, the compositions for use in the treatment of diseases of the posterior segment of the eye, by topical administration of any of dobesilic acid and or its salts (or

esters of any of them), are in a form selected from the group consisting of solutions, micellar solutions, gels, lotions, ointments, liposomes, suspensions, emulsions, nanoemulsions, microemulsions and creams.

With the aim of making such compositions easy to be administered by the patient, and also for avoiding disturbance of vision as much as possible when the compositions are applied, the compositions for use according to the invention are in a particular embodiment in the form of eye drop solutions. Even more in particular eye drop transparent solutions.

As will be depicted in the examples below, CDO and etamsylate administered as an eye drop liquid solution were able to pass through the cornea, sclera and/or conjunctiva, avoiding tear washing and finally reached the retina and the optic nerve, wherein high amounts of the drug could be found. This is of great relevance, because even with simple pharmaceutical compositions that are clear and transparent and thus maintain vision, the active principle can get target tissues.

Indeed, as demonstrated below, liquid pharmaceutical compositions comprising a therapeutically effective amount of dobesilic acid and/or another salt thereof, as well as an ester of the acid or the salt, a pH adjusting agent, optionally an antioxidant compound, and water, all properly sterilized (e.g. by UV means or sterilizing filtration) can be for use in the treatment and/or prevention of a disease of the posterior segment of the eye, wherein the treatment comprises administration of a topical dose of the dobesilic acid and/or a pharmaceutically acceptable salt, or ester of said acid or salt.

In another particular embodiment, optionally in combination with any embodiment above or below, the compositions for use in the treatment of diseases of the posterior segment of the eye, by administering topically onto eye surface a dose of the active principle, further comprise excipients selected from preservatives, pH adjusting agents, tonicity agents to adjust osmolality, chelating agents, penetration enhancers, bioadhesive polymers, stabilizers, viscosizing agents, tensioactives, antioxidants, and mixtures thereof.

Excipients used as pH adjusting agents are those allowing a pH from 3.8 to 8.6, more in particular from 4.5 to 8.0, even more in particular from 4.8 to 7.5, and preferably from 5.0 to 7.3, even more preferably from 5.5 to 6.9. Examples of pH adjusting agents include citrate salts (citric acid/citrate buffer), phosphate salts (phosporic acid/phosphate buffer), borate salts (boric acid/borate buffer), and mixtures thereof, all salts being those pharmaceuticaly accepatble. pH buffers may in additional comprise amino acids, in particular arginine, lysine, and an amine-derived compound selected from methylglucamine and trometamol, and mixtures thereof.

Thus, in a particular embodiment of the second aspect of the invention, the pharmaceutical topical compositions for use as above disclosed have a pH from 3.8 to 8.6, more in particular from 4.5 to 8.0, even more in particular from 4.8 to 7.5, and preferably from 5.0 to 7.3, even more preferably from 5.5 to 6.9.

The term "pH adjusting agent" refers to acids or bases or their mixtures that can be used to adjust the pH of the finished product to the desired level, without affecting the stability of the composition. In an embodiment, the composition for use according to the invention further comprises a pH adjusting agent selected from the group consisting of lactic acid and salts thereof (such as sodium lactate, potassium lactate and calcium lactate), citric acid and salts thereof (such as sodium citrate, potassium citrate, calcium citrate, lithium citrate, trisodium citrate and disodium hydrogen citrate), tartaric acid and salts thereof (such as sodium tartrate potassium tartrate, calcium tartrate and lithium tartrate), acetic acid and salts thereof (such as sodium acetate, potassium acetate and calcium acetate), hydrochloric acid, boric acid and salts thereof (sodium borate), sulphuric acid and salts thereof (such as sodium sulphate and potassium sulphate), nitric acid, hydrochloric acid, phosphoric acid and salts thereof (such as sodium dihydrogen phosphate, sodium monohydrogen phosphate, potassium dihidrogen phosphate lithium phosphate, potassium phosphate and calcium phosphate), carbonic acid and salts thereof (such as sodium carbonate, sodium hydrogen carbonate and potassium hydrogen carbonate), maleic acid and salts thereof (lithium maleate, sodium maleate, potassium maleate and calcium maleate), succinic acid and salts thereof (lithium succinate, sodium succinate, potassium succinate and calcium succinate), sodium hydroxide, potassium hydroxide, triethanolamine, diisopropanolamine, ammonia, tris(hydroxymethyl)aminomethane, tris(hydroxymethyl)aminomethane hydrochloride, and mixtures thereof. In an embodiment, the pH adjusting agent is selected from the group consisting of tris(hydroxymethyl)aminomethane, tris(hydroxymethyl)aminomethane hydrochloride, potassium dihydrogen phosphate, disodium hydrogen phosphate and mixtures thereof.

In another embodiment, the composition comprises a pH adjusting agent selected from the group consisting of acetic acid, boric acid, sorbic acid, citric acid, phosphoric acid, sodium phosphate, dibasic sodium phosphate, monobasic sodium phosphate, potassium dihydrogen phosphate and salts thereof, hydrochloric acid, sodium hydroxide, sodium thiosulfate, sodium sulfite, sodium sulphate, tris(hydroxymethyl)aminomethane, tris(hydroxymethyl)aminomethane hydrochloride, sodium hydrogen carbonate, sodium borate, sodium acetate, sodium bisulphate, sodium benzoate, sodium citrate and mixtures thereof.

Other components in the topical eye (ophthalmic) composition for the use according to the invention include, in particular embodiments, tensioactives, solvents (organic and inorganic solvents; i.e. water), viscosity agents (also named viscosizing agents), preservatives, tonicity agents, mucoadhesive polymers, chelating agents and enhancers of the active principle (i.e.: dobesilic acid and/or its salts or esters).

Among the tensoactives the non-ionic surfactants include, but are not limited to, ($C_{30}$-$C_{40}$)alkyl poly(ethylene oxide), block copolymers of poly(ethylene oxide) and poly(propylene oxide) (commercially called Poloxamers or Poloxamines), ($C_8$-$C_{14}$)alkyl polyglucosides including octyl glucoside and decyl maltoside, fatty alcoholsincluding cetyl alcohol and oleyl alcohol, cocamide MEA, cocamide DEA, sorbitan esters and derivatives thereof or sorbitan esters ethoxylate and derivatives thereof.

The viscosity agents are in particular polyvynil alcohol, compounds derived from cellulose such as methylcellulose hydroxymethylcellulose, hydroxyethylcellulose, sodium carboxy methylcellulose and hydroxypropylmethylcellulose, carbomers, polyethylenglicols, polyalcohols, chitosan, hyaluronic acid and mixtures thereof.

Examples of preservatives appropriate for the present invention include but is not limited to benzalkonium chloride, cetalkonium chloride, bezethonium chloride, chlorhexidine, benzyl alcohol, chlorobutanol, 2-phenylethanol,propylparaben, methylparaben, phenylmercuric acetate, phenylmercuric borate, sodiumdehydroacetate, sorbic acid phenylmercuric nitrate, cetyl pyridinium chloride, cetrimoniumbromide, benzyl bromide, sodium perborate, thimerosal and mixture thereof.

The tonicity agent is selected from the group consisting of sodium chloride, potassium chloride, calcium chloride, sodium phosphate, potassium phosphate, sodium hydrogen carbonate, calcium carbonate, sodium lactate, sorbitol, mannitol, xylitol, dextrose, polyethylene glycol, propylene glycol, dextran, and mixture thereof; preferably glycerin.

Agents enhancing absorption, also termed "enhancers" of the active ingredient are surfactants such as sorbitanmonolaurate, sorbitan monopalmitate, sorbitan trioleate, polyoxyethylene 20 sorbitan monolaurate, polyoxyethylene 20 sorbitan monopalmitate, polyoxyethylene 5 sorbitan monooleate, polyoxyethylene 20 sorbitan trioleate, polyoxyethylene 9 lauryl ether,polyoxyethylene 23 lauryl ether, polyoxyethylene 20 cetyl ether, polyoxyethylene 20 oleyl ether, polyethylene glycol octadecyl ether, polyoxyethylene 40 stearate, polyoxyethylene 50 stearate, palmitoyl carnitine, sodium caprate, stearylamine, sodium dodecyl sulfate, bile acids such as deoxycholic acid, taurocholic acid, taurodeoxycholic acid, urodeoxycholic acid, and tauroursodeoxycholic acid, fatty acids such as capric acid, caprylic and oleic acid, lauralkonium chloride, benzalkonium chloride, cetalkonium chloride, cetrimonium bromide , chlorhexidine digluconate, benzyl alcohol, chlorbutanol, 2-phenylethanol, paraben, propyl paraben and methyl paraben, EDTA, 1-dodecylazacycloheptan-2-one (Azone), hexamethylene lauramide, hexamethylene octanamide, decylmethylsulfoxide,saponin, cyclodextrins, pz-peptide, $\alpha$-amino acid, cetylpyridinium chloride, cytochalasins, ionophores or mixtures thereof

The mucoadhesive polymers, also referred as "bioadhesive polymers", refers to a substance which can increase residence time of the compositions of the invention. Examples of bioadhesive polymers appropriate for the present invention include polyvinylpirrolidones, such as Povidone K 17, Povidone K25, Povidone K 30 and Povidone K 90F; polyvinyl alcohol; xanthan gum; guar gum; welan gum; gellan gum; tragacanth gum; ceratonia gum; agar; methylcellulose; ethylcellulose; hydroxyethyl cellulose; hydroxyethylmethyl cellulose; hydroxypropyl cellulose; hydroxypropylmethyl cellulose; hydroxypropylmethyl cellulose phthalate; hydroxypropylmethyl cellulose acetate succinate; sodium carboxymethylcellulose; calcium carboxymethylcellulose; polyethylene glycol; glycerine; carrageenan; alginic acid; sodium alginate; potassium alginate; propylene glycol alginate; hyaluronic acid; sodium hyaluronate; poly(acrylic acid) derivatives such as carbomer and polycarbol; poloxamers; chitosan and chitosan derivatives; vinyl methyl ether/maleic anhydride copolymers; maltodextrin; polycaprolactone, and mixtures thereof.

The term "chelating agent" and "chelant" have the same meaning and are used interchangeable. They refer to a compound that is capable of complexing ions. Examples of chelating agents are citric acid, in particular citric acid monohydrate, EDTA (ethylenediaminetetraacetic acid) and its salts, such as dipotassium EDTA, disodium EDTA, calcium disodium EDTA, sodium EDTA and trisodium EDTA, fumaric acid, malic acid and maltol. In an embodiment, the chelating agent is selected from the group consisting of sodium edetate, citric acid, and salts and mixtures thereof.

Topical eye compositions of the present invention can be prepared according to methods well known in the state of the art. The appropriate excipients and/or carriers, as well as any pH adjusting agents, and their amounts, can readily be determined by those skilled in the art according to the type of formulation being prepared.

In a particular embodiment of the pharmaceutical topical eye composition for use as disclosed above, the composition is for immediate release of the dobesilic acid and/or pharmaceutically acceptable salt, or an ester of any of them. These types of formulations allow a quick release of the active ingredient serving for the purpose of rapidly acting on the different tissues of the posterior segment of the eye they finally reach.

On the alternative and in another particular embodiment, the pharmaceutical topical eye composition for use as defined above is a composition for the controlled along time release of the active principle, also termed modified-release composition or dosage forms. In particular, it is for the release of the active principle from 2 to 30 days. These compositions allow reducing the administration times, and promoting a simpler posology. Examples of these non-immediate release compositions, in which active principle is released during a period of time, comprise sustained-release compositions and prolonged release compositions. Sustained-release dosage forms are dosage forms designed to release (liberate) a drug at a predetermined rate in order to maintain a constant drug concentration for a specific period of time with minimum side effects. This can be achieved through a variety of formulations, including drug-polymer conjugates. Prolonged release compositions are compositions in which the drug is delivered within a period of time

(extended-release usually at a constant rate) or to a specific target in the body (targeted-release dosage).

For "release from 2 to 30 days", is to be understood when related to any of the aspects or embodiments of the invention, that the release of the dobesilic acid and/or a pharmaceutically acceptable salt, or an ester of any of the acid or the salt, is accomplished in such a way that at least 85 % of the active principle in the formulation has been released from the administration day to whatever day is the indicated for such formulation (e.g. 2, 5, 10, 20, or 30). That is, a pharmaceutical topical eye composition formulated for the release of the active principle in 2 days, means that at least 85%, more in particular 90%, and even more in particular 100% of the active principle has been released at day 2. In the same way, a release in 30 days means that at least 85%, more in particular 90%, and even more in particular 100% of the active principle has been released at day 30. The skilled man in the art of topical eye compositions will know the standard protocols for determining the release percentages of the active principle.

In another particular embodiment of the second aspect of the invention, the pharmaceutical topical eye compositions for use as disclosed above are formulated for administration to a subject, in particular a mammal, and more in particular a human, once a month.

In a more particular embodiment, the pharmaceutical topical eye compositions formulated for modified release administration comprises from 1 mg to 700 mg of the of dobesilic acid and/or of a pharmaceutically acceptable salt, or an ester of any of both of them.

In another particular embodiment of the second aspect of the invention, the pharmaceutical topical eye compositions for use as disclosed above are formulated for administration to a subject, in particular a mammal, and more in particular a human, once every 2, 4, 6, 8, 12 or 24 hours. This particular embodiment is more common in topical eye compositions for the proposed used that are in form of liquid compositions, suspensions or solutions, such as eye drop solutions, in which common posology includes application from one to several drops at predetermined intervals of time (e.g. two eye drops every 6 hours).

As indicated for the active ingredient, the pharmaceutical topical eye compositions for use as disclosed above are for the prevention and/or treatment of a disease selected from a retinal pathology, a vitreous humour pathology, a choroid pathology, an optic nerve pathology, posterior sclera pathologies and combinations thereof.

Indeed, the pharmaceutical topical compositions comprising a therapeutically effective amount of dobesilic acid and/or of a pharmaceutically acceptable salt, or an ester of any of the acid or the salt, are for use in a disease of the posterior segment of the eye, in which the vascular system of any of the tissues of this posterior segment is compromised. In particular, the vascular system in the retina (derived from the central retinal artery, also a branch of the ophthalmic artery, but passing in conjunction with the optic nerve) and the vascular system called uveal circulation (including along with the ciliary body and iris, the choroid).

In a particular embodiment, the pharmaceutical topical compositions are for use, according to the invention, in the treatment and/or prevention of the diseases listed above when referring to the dobesilic acid and/or salt, or esters thereof. Certain embodiments relate to to pharmaceutical topical eye compositions comprising a therapeutically effective amount of dobesilic acid and/or a pharmaceutically acceptable salt, or an ester of any of the acid or the salt, together with one or more pharmaceutically acceptable topical eye excipients and/or carriers, wherein the compositions are formulated for the release of dobesilic acid and/or a pharmaceutically acceptable salt, or an ester of any of the acid or the salt, from 2 to 30 days.

These pharmaceutical topical eye compositions according to the embodiments are modified-release compositions selected from sustained-release compositions and prolonged release compositions.

In a particular embodiment the composition comprises a therapeutically effective amount of dobesilic acid and/or a pharmaceutically acceptable salt, or an ester of any of the acid or the salt, from 1 mg to 700 mg. In yet another more particular embodiment, the composition comprises a therapeutically effective amount of dobesilic acid and/or a pharmaceutically acceptable salt, or an ester of any of the acid or the salt from 10 mg to 500 mg and it is formulated for the release of dobesilic acid and/or a pharmaceutically acceptable salt, or an ester of any of the acid or the salt, in 30 days.

In yet another particular embodiment the composition comprises a pharmaceutically acceptable salt of dobesilic acid, more in particular an alkali metal or alkaline earth metal salt of dobesilic acid. Even more in particular, it comprises calcium dobesilate. More in particular is CDO (calcium dobesilate monohydrate). Other pharmaceutical acceptable salts of dobesilic acid in another particular embodiment are selected from magnesium salt, potassium salt, sodium salt and mixtures thereof.

In another particular embodiment the pharmaceutically acceptable salt of dobesilic acid is a salt of dobesilic acid with an amine of formula (II), wherein $R^1$ and $R^2$ are equal and are ethyl radicals and $R^3$ is H. This salt of dobesilic acid is also known as etamsylate or as diethylamine 2,5-dihydroxybenzensulfonate. Hydrated salts of etamsylate are also used as ingredients in the topical eye compositions according to the embodiment

In another particular embodiment the composition further comprises excipients selected from preservatives, pH adjusting agents, tonicity agents to adjust osmolality, chelating agents, penetration enhancers, bioadhesive polymers, stabilizers, viscosizing agents, tensioactives, antioxidants, and mixtures thereof. Particular excipients and/or carriers disclosed and listed above when referring to the composition for use according to the second aspect of the invention,

are also excipients and/or carriers applicable to the pharmaceutical topical eye compositions of the embodiment

In a more particular embodiment the pharmaceutical topical eye compositions have a pH from 3.8 to 8.6, more in particular from 4.5 to 8.0, even more in particular from 4.8 to 7.5, and preferably from 5.0 to 7.3, even more preferably from 5.5 to 6.9. This pH is achieved with appropriate pH adjusting agents selected, in a particular embodiment, from the group consisting of acetic acid, boric acid, sorbic acid, citric acid, phosphoric acid, sodium phosphate, dibasic sodium phosphate, monobasic sodium phosphate, potassium dihydrogen phosphate and salts thereof, hydrochloric acid, sodium hydroxide, sodium thiosulfate, sodium sulfite, sodium sulphate, tris(hydroxymethyl)aminomethane, tris(hydroxymethyl)aminomethane hydrochloride, sodium hydrogen carbonate, sodium borate, sodium acetate, sodium bisulphate, sodium benzoate, sodium citrate and mixtures thereof.

In yet another particular embodiment optionally in combination with any embodiments above or below, the compositions further comprise a chelating agent, in particular selected from the group consisting of citric acid, in particular citric acid monohydrate, EDTA and its salts, such as dipotassium EDTA, disodium EDTA, calcium disodium EDTA, sodium EDTA and trisodium EDTA, fumaric acid, malic acid and maltol. In an embodiment, the chelating agent is selected from the group consisting of sodium edetate, citric acid, and salts and mixtures thereof.

In yet another particular embodiment optionally in combination with any embodiments above or below, the compositions further comprise an effective amount of a bioadhesive polymer. The bioadhesive polymers are in a particular embodiment selected from the group consisting of polyvinylpirrolidones, such as Povidone K 17, Povidone K25, Povidone K 30 and Povidone K 90F; polyvinyl alcohol; xanthan gum; guar gum; welan gum; gellan gum; tragacanth gum; ceratonia gum; agar;methylcellulose; ethylcellulose; hydroxyethyl cellulose; hydroxyethylmethyl cellulose; hydroxypropyl cellulose; hydroxypropylmethyl cellulose; hydroxypropylmethyl cellulose phthalate; hydroxypropylmethyl cellulose acetate succinate; sodium carboxymethylcellulose; calcium carboxymethylcellulose; polyethylene glycol; glycerine; carrageenan; alginic acid; sodium alginate; potassium alginate; propylene glycol alginate; hyaluronic acid; sodium hyaluronate; poly(acrylic acid) derivatives such as carbomer and polycarbol; poloxamers; chitosan and chitosan derivatives; vinyl methyl ether/maleic anhydride copolymers; maltodextrin; polycaprolactone, and mixtures thereof.

In yet another particular embodiment, the pharmaceutical topical eye compositions further comprise an effective amount of an antioxidant, in particular selected from the group consisting of butylated hydroxyanisole, butylated hydroxytoluene, tert-butylhydroquinone, tertiary butylhydroquinone, dilauryl thiopropionate, ethoxyquin, propyl gallate, 2,4,5-trihydroxybutyrophenone, thiodipropionic acid, alpha-tocopherol, ascorbic acid, sodium metabisulfite, sodium ascorbate, potassium metabisulfite, potassium sorbate, sodium benzoate, sodium iodide, sodium succinate hexahydrate, sodium sulfite, sodium tartrate, sodium thiosulfate, sorbic acid, trisodium citrate dihydrate, and mixtures thereof.

Optionally, the compositions comprise a pharmaceutically acceptable delivery system, which is a compound or mixture of compounds that preserve the active form of the dobesilic acid and/or pharmaceutically acceptable salt or ester thereof in the composition, and that allow the release of the dobesilic acid and/or a pharmaceutically acceptable salt, or an ester of any of the acid or the salt, to reach the posterior segment of the eye in an active form and in an effective amount.

Examples of these delivery systems comprise compounds that stabilize the active ingredient and or increase the residence time in the target tissues and reduce the elimination by the blood-retinal barrier.

Other pharmaceutical topical eye compositions according to the invention are those comprising:

- a therapeutically effective amount of dobesilic acid and/or a pharmaceutically acceptable salt, or an ester of any of the acid or the salt; and
- one or more pharmaceutically acceptable topical eye excipients and/or carriers, said excipients and/or carriers comprising a pH adjusting agent.

Particular embodiments of the compositions with pH adjusting agents comprise in such a category of agents a compound selected from the list indicated above for the second aspect of the invention. In a more particular embodiment, the topical eye composition comprises a pH adjusting agent selected from the group consisting of acetic acid, boric acid, sorbic acid, citric acid, phosphoric acid, sodium phosphate, dibasic sodium phosphate, monobasic sodium phosphate, potassium dihydrogen phosphate and salts thereof, hydrochloric acid, sodium hydroxide, sodium thiosulfate, sodium sulfite, sodium sulphate, tris(hydroxymethyl)aminomethane, tris(hydroxymethyl)aminomethane hydrochloride, sodium hydrogen carbonate, sodium borate, sodium acetate, sodium bisulphate, sodium benzoate, sodium citrate and mixtures thereof.

In a more particular embodiment of these compositions comprising the pH adjusting agent, they further comprise a chelant agent. Particular chelating agents are those listed for the second aspect the invention. More in particular they are selected from the group consisting of citric acid, in particular citric acid monohydrate, EDTA and its salts, such as dipotassium EDTA, disodium EDTA, calcium disodium EDTA, sodium EDTA and trisodium EDTA, fumaric acid, malic acid and maltol. In an embodiment, the chelating agent is selected from the group consisting of sodium edetate, citric acid, and salts and mixtures thereof.

In another particular embodiment, these compositions comprising the pH adjusting agent further comprise a further comprise an effective amount of a bioadhesive polymer. The bioadhesive polymers are in a particular embodiment selected from the group consisting of polyvinylpirrolidones, such as Povidone K 17, Povidone K25, Povidone K 30 and Povidone K 90F; polyvinyl alcohol; xanthan gum; guar gum; welan gum; gellan gum; tragacanth gum; ceratonia gum; agar;methylcellulose; ethylcellulose; hydroxyethyl cellulose; hydroxyethylmethyl cellulose; hydroxypropyl cellulose; hydroxypropylmethyl cellulose; hydroxypropylmethyl cellulose phthalate; hydroxypropylmethyl cellulose acetate succinate; sodium carboxymethylcellulose; calcium carboxymethylcellulose; polyethylene glycol; glycerine; carrageenan; alginic acid; sodium alginate; potassium alginate; propylene glycol alginate; hyaluronic acid; sodium hyaluronate; poly(acrylic acid) derivatives such as carbomer and polycarbol; poloxamers; chitosan and chitosan derivatives; vinyl methyl ether/maleic anhydride copolymers; maltodextrin; polycaprolactone, and mixtures thereof.

In yet another particular embodiment, the pharmaceutical topical eye compositions according with pH adjusting agents of the invention further comprise an effective amount of an antioxidant, in particular selected from the group consisting of butylated hydroxyanisole, butylated hydroxytoluene, tert-butylhydroquinone, tertiary butylhydroquinone, dilauryl thiopropionate, ethoxyquin, propyl gallate, 2,4,5-trihydroxybutyrophenone, thiodipropionic acid, alpha-tocopherol, ascorbic acid, sodium ascorbate, potassium metabisulfite, potassium sorbate, sodium benzoate, sodium iodide, sodium succinate hexahydrate, sodium sulfite, sodium tartrate, sodium thiosulfate, sorbic acid, trisodium citrate dihydrate, and mixtures thereof.

As indicated for the embodiments the pharmaceutical topical eye compositions comprising a pH adjusting agent also comprise, in another particular embodiment, a pharmaceutically acceptable delivery system as defined for the compositions of the embodiments

The invention relates also to alkali metal and alkaline earth metal salts or esters of dobesilic acid (i.e. calcium dobesilate, potassium dobesilate, sodium dobesilate, magnesium dobesilate) as such or in a pharmaceutical composition for use in the treatment and/or prevention of a disease of the posterior segment of the eye. In particular, for use in the treatment and/or prevention of a disease of the posterior segment of the eye selected from retinal and/or choroid pathologies, vitreous humour pathologies, optic nerve pathologies, posterior sclera pathologies and combinations thereof, wherein the treatment comprises administration of a topical dose onto eye surface of the alkali metal and alkaline earth metal salts or esters of dobesilic acid. In particular, it is for the treatment and/or prevention of any of the diseases listed above for any of the categories.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

## Examples

### Example 1. Compositions

Weigh the appropriate amount of CDO or etamsylate in a suitable vessel and add the distilled water referred in Table 1.

Dissolve the CDO or etamsylate in water with mild agitation.

Table 1.

| Composition | 1 | 2 | 3 | 4 | 5 | 6 | 7 |
|---|---|---|---|---|---|---|---|
| Calcium dobesilate monohydrate | 0.5 g | 1.0 g | 2.0 g | 7.5 g | 10.0 g | --- | --- |
| Etamsylate | --- | --- | --- | --- | --- | 1.26 g | 12.6 g |
| Distilled water | q.s. 100 ml | q.s. 100 ml | q.s. 100 ml | q.s. 100 ml | q.s. 100 ml | q.s. 100 ml | q.s. 100 ml |
| q.s. quantity sufficient for | | | | | | | |

**Example 2. Ocular distribution of CDO (topical ocular vs. oral single dose administrations) in rats and rabbits.**

**Methods**

Ocular distribution of CDO was determined in rat and rabbit animal models in order to compare bioavailability after oral administration and eye instillation.

*Ocular distribution of CDO in rats (topical ocular vs. oral administrations)*

In Sprague Dawley (SD) rat, weighing approximately 200 g, CDO was administered by topical ocular instillation (10 μl/eye) at 10 mg/ml (composition 2 of Example 1), at 100 mg/ml (composition 5 of Example 1) or by oral gavage at 50 mg/kg, at 200 mg/kg or at 750 mg/kg in aqueous solution (from compositions 1, 3 and 4, respectively, of Example 1). At 15, 60, 180 and 360 minutes post-administration, animals were euthanized and plasma and ocular tissues including sclera, cornea, retina, vitreous humour/lens and optic nerve were collected.

Oral dose of 50 mg/kg (10 mg) in rat was equivalent in human to one 500 mg capsule Doxium ®, 200 mg/kg (40 mg) to four 500 mg capsule Doxium ® dose and 750 mg/kg (150 mg) to fifteen 500 mg capsule Doxium ® based on the Human Equivalent Dose (HED) calculations (Guidance for Industry Estimating the Maximum Safe Starting Dose in Initial Clinical Trials for Therapeutics in Adult Healthy Volunteers U.S. Department of Health and Human Services Food and Drug Administration Center for Drug Evaluation and Research (CDER) July 2005 Pharmacology and Toxicology)

500 mg CDO Doxium ® (human weight 60 kg) = 8.3 mg CDO /kg human

HED (rat/human) = 6.2

8.3 x 6.2 = 51.5 mg/kg rat (rat weight 0.2 kg) =10 mg (dose equivalence in rat)

*Ocular distribution of CDO in rabbits (topical ocular vs. oral administration)*

In New Zealand White rabbit, weighing approximately 1.8 kg, CDO was administered by topical ocular instillation (33 μl/eye) at 10 mg/ml (composition 2 of Example 1), at 100 mg/ml (composition 5 of Example 1) or by oral gavage at 25 mg/kg in aqueous solution (from composition 1 of Example 1). At 15, 60, 180 and 360 minutes post-administration, animals were euthanized and plasma and ocular tissues including retina/choroid and optic nerve were collected.

Oral dose of 25 mg/kg (45mg) in rabbit corresponded to one 500 mg capsule Doxium ® in humans based on the HED calculations.

500 mg CDO Doxium ® (human weight 60 kg) = 8.3 mg CDO /kg human

HED (rabbit/human) = 3.1

8.3 x 3.1 = 25.7 mg/kg rabbit (rabbit weight 1.75 kg) =45 mg (dose equivalence in rabbit)

*Analytical conditions*

Bioanalysis of CDO was performed depending on the type of matrix. In the case of plasma, proteins were precipitated using methanol. In the case of tissues, samples were grinded in a Mini Bead Beater using zirconium beads and methanol/water (1:1) (v/v) as solvent. After centrifugation, samples were analyzed by UPLC-MS/MS.

Chromatographic separation was carried out using an Acquity CSH Fluoro Phenyl column (1.7 μm, 50 x 2.1 mm). Mobile phase consisted of a gradient using 0.1 % formic acid in water and acetonitrile from 5 % to 45 % in 4.5 min at a flow rate of 0.6 ml/min.

With regard to the mass spectrometric detection, the electrospray ionization source worked to record negative ions. Multiple Reaction Monitoring (MRM) acquisition mode of the deprotonated molecular ions was performed. The transition monitored for CDO was m/z 189.0 → 80.1 with a cone voltage of 45 V and collision energy of 25 eV.

**Results**

*Ocular distribution of CDO in rats (topical ocular vs. oral administrations)*

Next Table 2 shows concentration of CDO in µg/g of tissue at different test times:

Table 2.

| | CDO concentrations | | | | | | | | | | | |
|---|---|---|---|---|---|---|---|---|---|---|---|---|
| Admin. | Oral | | | | | | | | | | | |
| Dose | 50 mg/Kg = 10 mg | | | | 200 mg/Kg = 40 mg | | | | 750 mg/Kg = 150 mg | | | |
| Time (h) | 0.25 | 1 | 3 | 6 | 0.25 | 1 | 3 | 6 | 0.25 | 1 | 3 | 6 |
| Cornea (μg/g) | n.v | n.v v | n.v | n.v | n.v | n.v | n.v | n.v | n.v | n.v | n.v | n.v |
| Sclera (μg/g) | n.v | n. v | n.v | n.v | n.v | n.v | n.v | n.v | n.v | n.v | n.v | n.v |
| Retina (μg/g) | -- | -- | -- | -- | 0.11 | 0.47 | 1.04 | n.v | 0.67 | 1.34 | 2.42 | n.v |
| Optic nerve (μg/g) | -- | -- | 1.89 | 0.41 | n.v | n.v | n.v | n.v | n.v | n.v | n.v | n.v |
| Plasma (μg/ml) | n.v | n.v | n.v | n.v | 3.10 | 9.04 | 11.45 | n.v | 12.57 | 28.80 | 47.61 | n.v |

| Admin. | Topical eye administration | | | | | | | |
|---|---|---|---|---|---|---|---|---|
| Dose | 10 mg/ml (eye drop of 10 μl) = 0.1 mg | | | | 100 mg/ml (eye drop of 10 μl) = 1 mg | | | |
| Time (h) | 0.25 | 1 | 3 | 6 | 0.25 | 1 | 3 | 6 |
| Cornea (μg/g) | 4.61 | 1.01 | 1.99 | n.v | 58.02 | 1.72 | 3.11 | n.v |
| Sclera (μg/g) | 0.17 | 0.33 | 0.77 | n.v | 3.54 | 1.14 | 0.22 | n.v |
| Vitreous humour/ lens (μg/g) | 1.48 | 1.68 | 0.19 | n.v | n.v | n.v | n.v | n.v |
| Retina (μg/g) | 0.10 | -- | -- | -- | 2.03 | 0.64 | 0.25 | 0.13 |
| Retina/Choroid (μg/g) | 1.83 | 0.60 | 2.15 | n.v | 17.55 | 1.52 | 1.57 | n.v |
| Optic nerve (μg/g) | 5.30 | 0.85 | -- | 0.25 | 13.68 | 2.91 | 1.92 | 2.65 |
| Plasma (μg/ml) | 0.01 | 0.01 | 0.08 | n.v | 0.22 | 0.37 | 0.28 | n.v |

--: non-detectable
n.v: not measured
Abbreviation *Admin.* means administration

Data indicate that topical eye administration in rats leads to low plasmatic levels, which likely means that microvessels of anterior segment of the eye and the BRB in posterior segment avoid diffusion through the back of the eye to systemic circulation.

There is a fast absorption and distribution when CDO is topically applied onto eye surface.

Single dose (10 μl drop) topical ophthalmic administration in rats at 1% w/v (composition 2 of Example 1) allows detection of CDO in optic nerve.

There is a better bioavailability following the ophthalmic topical route, since 1 mg CDO instilled onto the eye surface show equivalent levels in retina to 40-150 mg of CDO orally administered.

*Ocular distribution of CDO in rabbits (topical ocular vs. oral administration)*

Next Table 3 shows concentration of CDO in μg/g of tissue at different test times:

Table 3.

| | CDO μg/g tissue | | | |
|---|---|---|---|---|
| Administration | Oral 25 mg/Kg = 45 mg | | | |
| Time (h) | 0.25 | 1 | 3 | 6 |
| Retina/Choroid | 0.43 | 0.95 | 0.28 | 0.12 |
| Optic nerve | 0.74 | 1.61 | 0.49 | 0.10 |
| Administration | Topical eye administration 10 mg/ml = 0.3 mg | | | |
| Time (h) | 0.25 | 1 | 3 | 6 |
| Retina/Choroid | 0.27 | 0.29 | 0.16 | 0.11 |
| Optic nerve | 0.23 | 0.76 | 0.65 | 0.45 |
| Administration | Topical eye administration 100 mg/ml = 3.3 mg | | | |
| Time (h) | 0.25 | 1 | 3 | 6 |
| Retina/Choroid | 7.88 | 6.41 | 3.47 | 1.14 |
| Optic nerve | 14.39 | 22.91 | 4.50 | 1.92 |

Data indicate that topical eye administration in rabbits (single low dose 0.3 mg) gives values of CDO in target tissues equivalent to those of a single oral dose (45 mg). If a single dose with higher amounts (CDO 3.3 mg) are applied topically onto the eye, higher levels of the drug are detected than those obtained following a single oral dose. Again, it was demonstrated that CDO topically applied onto the eye surface shows a high bioavailability.

Furthermore CDO amounts could be detected in the target tissues 6 hours after topical ocular administration in particular in the optic nerve and retina choroids in both animal models.

**Example 3. Ocular distribution of etamsylate following topical ocular single dose administrations in rats.**

**Methods**

Ocular distribution of etamsylate was determined in rat in order to confirm the absorption of the drug into the posterior segment of the eye.

In Sprague Dawley (SD) rat, weighing approximately 200 g, etamsylate was administered by topical ocular instillation (10 μl/eye) compositions 6 and 7 of Example 1. At 15, 60 and 180 minutes post-administration, animals were euthanized and ocular tissues including retina, and optic nerve were collected.

*Analytical conditions*

As described in Example 2.

**Results**

Next table 4 shows the experimental results:

Table 4.

| | | CDO μg/g tissue | |
|---|---|---|---|
| Tissue | Time (h) | Topical eye administration single dose (10μl/eye) | |
| | | 12.6 mg/ml = 0.126 mg | 126 mg/ml = 1,26 mg |
| Retina | 0.25 | 4.39 | 4.05 |
| | 1 | 3.48 | 2.42 |
| | 3 | 0.27 | 1.56 |
| Optic nerve | 0.25 | 36.46 | 25.17 |
| | 1 | 8.13 | 15.79 |
| | 3 | 2.32 | 2.67 |

The absorption of another dobesilic acid salt (etamsylate) is confirmed. The active ingredient reaches the posterior eye segment of the eye, in particular retina and optic nerve in high amounts when applied topically onto eye surface (eye drops onto cornea and conjunctiva).

These data altogether allow concluding that dobesilic acid in form of a salt is absorbed and quickly transported to the posterior segment of the eye structures independently of the salt.

**Example 4. Ocular distribution of CDO following topical ocular repeated dose administrations in rats.**

**Methods**

28-day repeated dose study in the diabetic rats was performed to assess CDO levels in retina at the steady state. CDO compositions 2 and 5 of Example 1 were administered by topical ocular instillation (10 μl/eye) TID for 28 days. At day 29 CDO was instilled a single time and retinas were collected 15 minutes post-administration.

*Analytical conditions*

As described in Example 2.

**Results**

Next Table 5 shows concentration of CDO in μg/g of retinal tissue measured after 28-day repeated dose study; and measured at day 29, 15 minutes after single dose administration of CDO.

Table 5

| | | CDO μg/g tissue | |
|---|---|---|---|
| Tissue | Time (h) | Topical eye administration 28 days (3 administrations/day, 10μl/eye) | |
| | | 10 mg/ml | 100 mg/ml |
| Retina | 0.25 | 19.5 | 218.0 |

The above summarized data confirms the right absorption and distribution of CDO after topical ocular administration and the elevated concentration of the drug in the retinal tissues. A good correlation is as well observed between dose and concentration of CDO in retina in the steady state study. Repeated dose reinforce the data obtained after single dose administration.

**Example 5. Efficacy of CDO in Diabetic retinopathy model induced by streptozotocin in rat**

**Methods**

Diabetes was induced in SD male rats by single intraperitoneal injection of streptozotocin (STZ) at 60 mg/kg body weight in citrate buffer (pH 4.5). Seven days after STZ administration, blood glucose was measured and the level of 250 mg/dl was considered the threshold value to consider diabetes induction. Ophthalmic treatment with CDO at 10 mg/ml (topical instillation of 10 μl/eye) was initiated once hyperglycemia was confirmed and continued for 28 days three times a day (TID). Rats were randomly divided in 3 groups: I. Control (non-diabetic), II. Diabetic rats with no treatment and III. Diabetic rats with CDO at 10 mg/ml.

At day 29 CDO was instilled a single time at 10 mg/ml in the group III, rats were euthanized and retinas were collected 15 minutes post-administration.

*Retinal vascularity*

Retinal vascularity was observed by Evans Blue (EB) staining, based on the ability of EB to bind serum albumin immediately after intravenous injection. Thus Evans Blue 3% was injected through the tail vein at 1 ml/kg at 60 minutes before euthanasia at day 29. Eyeballs were fixed in paraformaldehyde 4%. Retinas were immediately after dissected and mounted in slides and observed by fluorescence microscopy.

*Antioxidant capacity*

The antioxidant capacity was evaluated by determining the concentrations of Glutathione disulfide (GSSG) or oxidized glutathione in the homogenized retinas. Tissues were collected at day 29 and homogenized in PBS pH 6-7 containing 1 mM EDTA. After centrifugation at 10000 rpm, an aliquot of the supernatant was diluted in 0.1 % formic acid and analyzed by UPLC-MS/MS.

Chromatographic analysis was carried out using an Acquity HSST3 column (1.8 μm, 50 x 2.1 mm). Isocratic elution was performed at a flow rate of 0.6 ml/min with a mixture of 0.1 % formic acid and methanol (99:1) as mobile phase.

With regard to the mass spectrometric detection, the electrospray ionization source worked to record negative ions. Multiple Reaction Monitoring acquisition mode of the deprotonated molecular ions was performed. The transition monitored for GSSG was m/z 611.2 → 306.2 with a cone voltage of 45 V and collision energy of 25 eV.

*Anti-inflammatory activity*

RNA was isolated from each retina by using the commercial kit SV Total RNA Isolation System (Promega) and stored at -80°C until use. Relative mRNA levels were quantified using a MX3000 (Stratagene) quantitative PCR system. Reverse transcription and cDNA amplification were performed using One-Step qRT-PCR SYBR Green PCR Master Mix with target specific primers for genes. The thermocycler parameters were one cycle of 50°C for 15 minutes; 95°C for 2 minutes followed by 40 cycles of 95°C for 30 seconds, 55°C for 30 seconds and 68°C for 30 seconds and finally 1 cycle of 95°C for 60 seconds, 55°C for 30 seconds and 95°C for 30 seconds.

Primer sequences were the following:

β-actin: (Forward primer, 5'- GTCGTACCACTGGCATTGTG -3' (SEQ ID NO: 1); and Reverse primer, 5'- CTCTCAGCTGTGGTGGTGAA -3' (SEQ ID NO: 2)).

TNF-α: (Forward primer, 5'- ATCTACTCCAGGTCCTCTTC -3' (SEQ ID NO: 3); and Reverse primer, 5'- GATGCGGCTGATGGTGTG -3' (SEQ ID NO: 4)).

The qPCR system detects the fluorescence emitted by SYBR, its increase corresponds to exponential increase of the PCR product. The software calculates the threshold cycle (Ct), in which the fluorescence exceeds the background signal. Relative changes in gene expression were calculated using the ΔCt method. The amount of gene expression was normalized to β-actin as housekeeping gene. Relative expression of mRNA in the test samples were determined as fold increase compared with the control sample (non-diabetic).

**Results**

*Retinal vascularity:*

Retinas were assessed by fluorescence microscopy. In diabetic rats (not treated group II) the retinal vascularity was clearly impaired and microvascular lesions were observed being consistent with the early stages of diabetic retinopathy. It was observed an increased permeability from the capillaries as well as an evident retinal vessel leakage. Non severe angiogenesis processes were observed in this diabetic non treated group II. The diabetic rats (group III) treated with CDO at 10 mg/ml exhibited well preserved retinas, with well-defined vessels with no evidence of vascular damages. The overall appearance of the retinas was closer to the no diabetic control group I.

*Antioxidant capacity:*

Concentrations of GSSG determined in the homogenized retinas of the diabetic rats (not treated group II and treated group III) as well as the control group I are shown in figure 1. The retinal concentrations of GSSG for diabetic rats (not treated) were higher than those for the control group) ($P<0.05$, One way ANOVA with Bonferroni correction).

Nevertheless, the diabetic rats treated with CDO at 10 mg/ml were able to return concentrations of GSSG at the same level as the control group ($P<0.01$ group II vs. group III, One way ANOVA with Bonferroni correction), demonstrating the antioxidant capacity of CDO in this rat diabetic retinopathy model. These results confirm the activity of CDO in the target tissue and the significant prevention of the reactive oxygen species generation related to the oxidative stress processes induced by high levels of glycaemia.

*Anti-inflammatory activity*

Relative expression of TNF-$\alpha$ mRNA in retinas of diabetic rats (non-treated group II and treated group III) as well as the control group I (non-diabetic) is shown in figure 2. It was observed an increase of the inflammatory cytokine TNF-$\alpha$ level in the retinas of diabetic group II. The treatment with CDO at 10 mg/ml was able to significantly decrease the TNF-$\alpha$ levels ($P<0.01$ group II vs. group III, One way ANOVA with Bonferroni correction), furthermore the expression of the treated group was slightly lower than the control group, demonstrating the anti-inflammatory effect of CDO in this rat diabetic retinopathy model.

**Example 6. Ocular Irritation Assay (HET-CAM)**

**Methods**

The HET-CAM is an alternative method to the Draize Rabbit Eye Test which mimics vascular changes in the chorioallantoic membrane, an analog for ocular conjunctiva, which can be used to determine the potential irritancy of a test substance. This method is based on that described in Hen's Egg Test - Chorioallantoic Membrane (HET-CAM) Test Method (ICCVAM-Recommended Test Method Protocol, NIH Publication No. 10-7553 - Published 2010)

Fertilized white SPF (special pathogen free) White Leghorn chicken eggs were incubated at 37 °C and 60 % humidity up to day 10. After determination of the viability of the embryo, a rectangular window was removed from the shell directly over the air cell and the egg membrane was carefully moistened with 2-3 ml 0.9 % saline. After returning to the incubator for 30 minutes, the inner membrane was removed and the test substance was applied to the CAM membrane with a pipette. Eggs were observed continuously for 5 minutes for the appearance of lysis, hemorrhage and/or coagulation and an irritation score (IS) was determined following the ICCVAM-Recommended Test Method Protocol (NIH Publication No. 10-7553-Published 2010) guideline.

**Results**

HET-CAM ocular irritation assay was performed and representative images of NaCl 0.9% (negative control), NaOH 0.1% (positive control), etamsylate (100 mg/ml), potassium dobesilate (100 mg/ml) and CDO (100 mg/ml) are shown in figure 3. The assayed active ingredients did not trigger any lysis, haemorrhage or coagulation processes and they consequently were characterized as not irritating. IS is the irritation score.

**Example 7. Ocular tolerability of CDO in rats.**

**Methods**

Diabetic SD male rats were treated with CDO compositions 2 and 5 of Example 1 for 28 days three times a day (topical instillation of 10μl/eye). Clinical evaluation of the eye appearance was performed thorough the study.

**Results**

The clinical eye evaluation of the treated rats did not reveal irritation symptoms, indicating a good tolerance of the assayed compositions.

**Citation List**

Patent Literature

- EP2875811
- EP2777699

Non Patent Literature

- Awwad et al., "Principles of Pharmacology in the Eye", British Journal of Pharmacology-2017, vol no. 174 Issue 23, pp.:4205-4223.
- Cuevas et al., "Single Intravitreal Injection of Etamsylate for the Treatment of Geographic Atrophy Associated with Submacular Hemorrhage", MOJ Clin Med Case - 2017, vol. no.7(1): 00186.
- Cuevas et al., "Intravitreal Dobesilate Injection for Macular Oedema Secondary to Branch Retinal Vein Occlusion", MOJ Clin Med Case - 2016, vol. no.4(1): 00078.
- ICCVAM-Recommended Test Method Protocol, NIH Publication No. 10-7553 - Published 2010
- Gaudana et al, "Ocular drug delivery", American Association of Pharmaceutical Sci. 2010 Sep;12(3):348-360. doi: 10.1208/s12248-010-9183-3. Epub 2010 May 1.
- Vadlapudi et al, "Ocular Drug Delivery", Chapter 10, pp.:2019-263.
- Simó et al., "Mechanisms of retinal neuroprotection of calcium dobesilate: therapeutic implications" Neural Regen Res -2017, vol. no.12(10), pp.:1620-1622.
- del Amo et al., "Current and future ophthalmic drug delivery systems. A shift to the posterior segment", Drug Discovery Today- 2008, vol. no. 13(3/4), pp.: 135-143
- Guidance for Industry Estimating the Maximum Safe Starting Dose in Initial Clinical Trials for Therapeutics in Adult Healthy Volunteers U.S. Department of Health and Human Services Food and Drug Administration Center for Drug Evaluation and Research (CDER) July 2005 Pharmacology and Toxicology.
- Ranta et al., "Barrier analysis of periocular drug delivery to theposterior segment", Journal of Controlled Release-2010, vol. no. 148, pp.: 42-48.


**Claims**

1. Dobesilic acid and/or a pharmaceutically acceptable salt, or an ester of any of the acid or the salt, for use in the treatment and/or prevention of a disease of the posterior segment of the eye, wherein the treatment comprises administration of a topical dose of the dobesilic acid and/or a pharmaceutically acceptable salt, or ester of said acid or salt, from 0.01 mg/day to 400 mg/day.

2. The dobesilic acid and/or a pharmaceutically acceptable salt, or an ester of any of the acid or the salt for use according to claim 1, wherein the pharmaceutically acceptable salt is selected from an alkali metal salt of dobesilic acid, an alkaline earth metal salt of dobesilic acid, a salt of dobesilic acid with an amine of formula (II), and combinations thereof

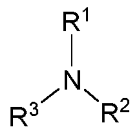 (II),

wherein $R^1$, $R^2$ and $R^3$ are independently selected from -($C_1$-$C_8$)-alkyl radicals or H.

**3.** The dobesilic acid and/or a pharmaceutically acceptable salt, or an ester of any of the acid or the salt, for use according to any of claims 1-2, which is a pharmaceutically acceptable salt selected from an alkali metal salt of dobesilic acid, an alkaline earth metal salt of dobesilic acid, and combinations thereof.

**4.** The dobesilic acid and/or a pharmaceutically acceptable salt, or an ester of any of the acid or the salt, for use according to any of claims 1-3, which is the alkaline earth metal salt of dobesilic acid calcium dobesilate.

**5.** The dobesilic acid and/or a pharmaceutically acceptable salt, or an ester of any of the acid or the salt, for use according to any of claims 1-2, which is the salt of dobesilic acid with an amine of formula (II), wherein $R^1$ and $R^2$ are equal and are ethyl radicals.

**6.** The dobesilic acid and/or a pharmaceutically acceptable salt, or an ester of any of the acid or the salt, for use according to any of claims 1-5, wherein the disease of the posterior segment of the eye is selected from retinal and/or choroid pathologies, vitreous humour pathologies, posterior sclera pathology, optic nerve pathologies, and combinations thereof.

**7.** The dobesilic acid and/or a pharmaceutically acceptable salt, or an ester of any of the acid or the salt, for use according to any of claims 1-6, wherein the disease of the posterior segment of the eye is a retinal pathology and/or choroid pathology selected from a retinal vasculopathy, a maculopathy, an hereditary eye fundus dystrophy, an idiopatic chorioretinopathy, a central serous retinopathy, generalized choroidal dystrophy, and combinations thereof.

**8.** The dobesilic acid and/or a pharmaceutically acceptable salt, or an ester of any of the acid or the salt thereof for use according to claim 7, wherein the retinal vasculopathy is selected from diabetic retinopathy, diabetic papillopathy, non-diabetic retinopathy, ocular ischemic syndrome, hypertensive retinopathy, thalassemia retinopathy, Coats' syndrome, Eales' syndrome, radiation retinopathy, solar retinopathy, purtscher retinopathy, polypoidal choroidal vasculopathy (PCV), retinal macroaneurysm, retinal microaneurysm, leukemic retinopathy, retinal ischemia, chronic retina disorders, and combinations thereof.

**9.** The dobesilic acid and/or a pharmaceutically acceptable salt, or an ester of any of the acid or the salt for use according to claim 7, wherein the maculopathy is selected from aged-related macular degeneration (ARMD), hemorrhagic ARMD, retinal angiomatous proliferation, polipoidal choroidal vasculopathy, malattia leventinese, full thickness macular hole, macular epiretinal membrane, macular telangiectasias, cellophane maculopathy or macular pucker, myopia maculopathy, exudative maculopathy after venous thrombosis of the retina, acute macular neuroretinopathy, macular cystoids, macular edema, retinal angioid streaks, choroidal folds, hypotony maculopathy and combinations thereof; and wherein the hereditary eye fundus distrophy is selected from the group consisting of retinitis pigmentosa; atypical retinitis pigmentosa including but not limited to Usher's syndrome, retinitis punctata albicans, Leber's congenital amaurosis, dystrophy of the cones, rod dystrophy, Bietti crystalline corneoretinal dystrophy, juvenile macular dystrophy, all types of macular dystrophy, Stargardt's disease or Fundus flavimaculatus, Usher's Syndrome and combinations thereof.

**10.** The dobesilic acid and/or a pharmaceutically acceptable salt, or an ester of any of the acid or the salt for use according to any of claims 1-6, wherein the disease of the posterior eye segment is a vitreous humour pathology selected from the group consisting of sub-macular and vitreous haemorrhages, asteroid hyalosis, vitreous detachment, eye floaters or myodesopsia, hereditary vitreoretinopathies, Stickler's syndrome or Wagner's Syndrome, and combinations thereof.

**11.** The dobesilic acid and/or a pharmaceutically acceptable salt, or an ester of any of the acid or the salt for use according to any of claims 1-6, wherein the disease of the posterior segment of the eye is an optic nerve pathology selected from the group consisting of optic atrophy, optic neuritis, neuroretinitis, ischemic neuropathy, hereditary optic neuropathy, toxic amblyopia or nutritional optic neuropathy, ocular hypertension, primary glaucoma , secondary

glaucoma, iridocorneal endothelial syndrome associated to glaucoma, optic nerve head drusen or optic disc drusen, papilledema and combinations thereof.

**12.** A pharmaceutical topical eye composition for use in the treatment and/or prevention of a disease of the posterior segment of the eye, which comprises a therapeutically effective amount of dobesilic acid and/or of a pharmaceutically acceptable salt, or an ester of any of the acid or the salt, together with one or more pharmaceutically acceptable topical excipients and/or carriers; wherein the treatment comprises administration of a topical dose of the dobesilic acid and/or a pharmaceutically acceptable salt, or an ester thereof, from 0.01 mg/day to 400 mg/day.

**13.** The pharmaceutical topical eye composition for use according to claim 12, comprising dobesilic acid and/or a pharmaceutically acceptable salt, or an ester of any of the acid or the salt, in a percentage by weight/volume from 0.02 % to 20% w/v.

**14.** The pharmaceutical topical eye composition for use according to any of claims 12-13, wherein the pharmaceutically acceptable salt is selected from an alkali metal salt of dobesilic acid, an alkaline earth metal salt of dobesilic acid, a salt of dobesilic acid with an amine of formula (II), and combinations thereof:

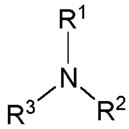 (II),

wherein $R^1$, $R^2$ and $R^3$ are independently selected from -($C_1$-$C_8$)-alkyl radicals or H.

**15.** The pharmaceutical topical eye composition for use according to any of claims 12-14, wherein the pharmaceutically acceptable salt is the alkaline earth metal salt of dobesilic acid calcium dobesilate.

## Patentansprüche

**1.** Dobesilsäure und/oder ein pharmazeutisch akzeptables Salz, oder ein Ester der Säure oder des Salzes, zur Verwendung bei der Behandlung und/oder Prävention einer Erkrankung des hinteren Augenabschnitts, wobei die Behandlung die Verabreichung von einer topischen Dosis der Dobesilsäure und/oder eines pharmazeutisch akzeptablen Salzes, oder Esters der Säure oder des Salzes, von 0,01 mg/Tag bis 400 mg/Tag umfasst.

**2.** Die Dobesilsäure und/oder ein pharmazeutisch akzeptables Salz, oder ein Ester der Säure oder des Salzes zur Verwendung nach Anspruch 1, wobei das pharmazeutisch akzeptable Salz ausgewählt ist aus einem Alkalimetallsalz von Dobesilsäure, einem Erdalkalimetallsalz von Dobesilsäure, einem Salz von Dobesilsäure mit einem Amin der Formel (II) und Kombinationen davon

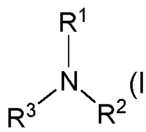 (II),

wobei $R^1$, $R^2$ und $R^3$ unabhängig voneinander ausgewählt sind aus -($C_1$-$C_8$)-Alkylresten oder H.

**3.** Die Dobesilsäure und/oder ein pharmazeutisch akzeptables Salz, oder ein Ester der Säure oder des Salzes, zur Verwendung nach einem der Ansprüche 1 bis 2, welches ein pharmazeutisch akzeptables Salz ist, das ausgewählt ist aus einem Alkalimetallsalz von Dobesilsäure, einem Erdalkalimetallsalz von Dobesilsäure und Kombinationen davon.

**4.** Die Dobesilsäure und/oder ein pharmazeutisch akzeptables Salz, oder ein Ester der Säure oder des Salzes, zur Verwendung nach einem der Ansprüche 1 bis 3, welches das Erdalkalimetallsalz von Dobesilsäure Calciumdobesilat ist.

**5.** Die Dobesilsäure und/oder ein pharmazeutisch akzeptables Salz, oder ein Ester der Säure oder des Salzes, zur Verwendung nach einem der Ansprüche 1 bis 2, welches das Salz von Dobesilsäure mit einem Amin der Formel (II) ist, wobei $R^1$ und $R^2$ gleich sind und Ethylreste sind.

**6.** Die Dobesilsäure und/oder ein pharmazeutisch akzeptables Salz, oder ein Ester der Säure oder des Salzes, zur Verwendung nach einem der Ansprüche 1 bis 5, wobei die Erkrankung des hinteren Augenabschnitts ausgewählt ist aus Netzhaut- und/oder Aderhautpathologien, Glaskörperpathologien, Pathologien der hinteren Sklera, Sehnervenpathologien und Kombinationen davon.

**7.** Die Dobesilsäure und/oder ein pharmazeutisch akzeptables Salz, oder ein Ester der Säure oder des Salzes, zur Verwendung nach einem der Ansprüche 1 bis 6, wobei die Erkrankung des hinteren Augenabschnitts eine Netzhautpathologie und/oder eine Aderhautpathologie ist, die ausgewählt ist aus einer retinalen Vaskulopathie, einer Makulopathie, einer erblichen Augenhintergrunddystrophie, einer idiopatischen Chorioretinopathie, einer zentralen serösen Retinopathie, einer generalisierten Aderhautdystrophie und Kombinationen davon.

**8.** Die Dobesilsäure und/oder ein pharmazeutisch akzeptables Salz, oder ein Ester der Säure oder des Salzes davon zur Verwendung nach Anspruch 7, wobei die retinale Vaskulopathie ausgewählt ist aus diabetischer Retinopathie, diabetischer Papillopathie, nicht-diabetischer Retinopathie, okularem ischämischem Syndrom, hypertensiver Retinopathie, Thalassämie-Retinopathie, Coats-Syndrom, Eales-Syndrom, Strahlenretinopathie, solarer Retinopathie, Purtscher-Retinopathie, polypoidaler choroidaler Vaskulopathie (PCV), retinalem Makroaneurysma, retinalem Mikroaneurysma, leukämischer Retinopathie, Netzhautischämie, chronischen Netzhauterkrankungen, und Kombinationen davon.

**9.** Die Dobesilsäure und/oder ein pharmazeutisch akzeptables Salz, oder ein Ester der Säure oder des Salzes zur Verwendung nach Anspruch 7, wobei die Makulopathie ausgewählt ist aus altersbedingter Makuladegeneration (AMD), hämorrhagischer AMD, retinaler angiomatöser Proliferation, polipoider choroidaler Vaskulopathie, Malattia Leventinese, Makulaloch in voller Dicke, epiretinaler Makulamembran, makulären Teleangiektasien, Zellophan-Makulopathie oder Makula-Pucker, Myopie-Makulopathie, exsudativer Makulopathie nach venöser Thrombose der Netzhaut, akuter makulärer Neuroretinopathie, makulären Zystoiden, Makulaödem, retinalen angioiden Streaks, Aderhautfalten, Hypotonie-Makulopathie und Kombinationen davon; und wobei die erbliche Augenhintergrunddystrophie ausgewählt ist aus der Gruppe bestehend aus Retinitis pigmentosa; atypischer Retinitis pigmentosa, einschließlich, aber nicht beschränkt auf Usher-Syndrom, Retinitis punctata albicans, Leberscher angeborener Amaurose, Zapfendystrophie, Stäbchendystrophie, Bietti-kristalliner korneoretinaler Dystrophie, juveniler makulärer Dystrophie, allen Arten von makulärer Dystrophie, Morbus Stargardt oder Fundus flavimaculatus, Usher-Syndrom und Kombinationen davon.

**10.** Die Dobesilsäure und/oder ein pharmazeutisch akzeptables Salz, oder ein Ester der Säure oder des Salzes zur Verwendung nach einem der Ansprüche 1 bis 6, wobei die Erkrankung des hinteren Augensegments eine Glaskörperpathologie ist, die ausgewählt ist aus der Gruppe bestehend aus submakulären Blutungen und Glaskörperblutungen, Asteroidenhyalose, Glaskörperablösung, *Mouches volantes* oder Myodesopsie, erblichen Vitreoretinopathien, Stickler-Syndrom oder Wagner-Syndrom und Kombinationen davon.

**11.** Die Dobesilsäure und/oder ein pharmazeutisch akzeptables Salz, oder ein Ester der Säure oder des Salzes zur Verwendung nach einem der Ansprüche 1 bis 6, wobei die Erkrankung des hinteren Augenabschnitts eine Sehnervenpathologie ist, die ausgewählt ist aus der Gruppe bestehend aus Optikusatrophie, Optikusneuritis, Neuroretinitis, ischämischer Neuropathie, erblicher Optikusneuropathie, toxischer Amblyopie oder ernährungsbedingter Optikusneuropathie, okulärer Hypertonie, primärem Glaukom, sekundärem Glaukom, iridokornealem Endothel-Syndrom in Verbindung mit Glaukom, Drusenpapille oder Drusen der Papilla nervi optici, Papillenödem und Kombinationen davon.

**12.** Eine pharmazeutische topische Augenzusammensetzung zur Verwendung bei der Behandlung und/oder Prävention einer Erkrankung des hinteren Augenabschnitts, welche eine therapeutisch wirksame Menge an Dobesilsäure und/oder eines pharmazeutisch akzeptablen Salzes, oder eines Ester der Säure oder des Salzes, zusammen mit einem oder mehreren pharmazeutisch akzeptablen topischen Hilfsstoffen und/oder Trägersubstanzen umfasst; wobei die Behandlung die Verabreichung einer topischen Dosis der Dobesilsäure und/oder eines pharmazeutisch akzeptablen Salzes, oder eines Esters davon, von 0,01 mg/Tag bis 400 mg/Tag umfasst.

**13.** Die pharmazeutische topische Augenzusammensetzung zur Verwendung nach Anspruch 12, umfassend Dobesilsäure und/oder ein pharmazeutisch akzeptables Salz, oder ein Ester der Säure oder des Salzes, in einem Gewichts-/Volumenprozentsatz von 0,02 % bis 20 % w/v.

14. Die pharmazeutische topische Augenzusammensetzung zur Verwendung nach einem der Ansprüche 12 bis 13, wobei das pharmazeutisch akzeptable Salz ausgewählt ist aus einem Alkalimetallsalz von Dobesilsäure, einem Erdalkalimetallsalz von Dobesilsäure, einem Salz von Dobesilsäure mit einem Amin der Formel (II), und Kombinationen davon:

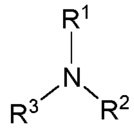

(II),

wobei $R^1$, $R^2$ und $R^3$ unabhängig voneinander ausgewählt sind aus -($C_1$-$C_8$)-Alkylresten oder H.

15. Die pharmazeutische topische Augenzusammensetzung zur Verwendung nach einem der Ansprüche 12 bis 14, wobei das pharmazeutisch akzeptable Salz das Erdalkalimetallsalz von Dobesilsäure Calciumdobesilat ist.

**Revendications**

1. Acide dobésilique et/ou un sel pharmaceutiquement acceptable, ou un ester de l'un quelconque de l'acide ou du sel, pour l'utilisation dans le traitement et/ou la prévention d'une maladie du segment postérieur de l'œil, dans lequel le traitement comprend l'administration d'une dose topique de l'acide dobésilique et/ou d'un sel pharmaceutiquement acceptable, ou ester dudit acide ou sel, de 0,01 mg/jour à 400 mg/jour.

2. L'acide dobésilique et/ou un sel pharmaceutiquement acceptable, ou un ester de l'un quelconque de l'acide ou du sel, pour l'utilisation selon la revendication 1, dans lequel le sel pharmaceutiquement acceptable est choisi parmi un sel de métal alcalin de l'acide dobésilique, un sel de métal alcalino-terreux de l'acide dobésilique, un sel de l'acide dobésilique avec une amine de formule (II), et des combinaisons de ceux-ci

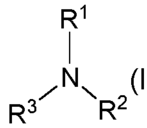 (II),

dans laquelle $R^1$, $R^2$et $R^3$ sont indépendamment choisis parmi des radicaux alkyle en-($C_1$-$C_8$)- ou H.

3. L'acide dobésilique et/ou un sel pharmaceutiquement acceptable, ou un ester de l'un quelconque de l'acide ou du sel, pour l'utilisation selon l'une quelconque des revendications 1 à 2, qui est un sel pharmaceutiquement acceptable choisi parmi un sel de métal alcalin de l'acide dobésilique, un sel de métal alcalino-terreux de l'acide dobésilique, et des combinaisons de ceux-ci.

4. L'acide dobésilique et/ou un sel pharmaceutiquement acceptable, ou un ester de l'un quelconque de l'acide ou du sel, pour l'utilisation selon l'une quelconque des revendications 1 à 3, qui est le sel de métal alcalino-terreux de l'acide dobésilique dobésilate de calcium.

5. L'acide dobésilique et/ou un sel pharmaceutiquement acceptable, ou un ester de l'un quelconque de l'acide ou du sel, pour l'utilisation selon l'une quelconque des revendications 1 à 2, qui est le sel de l'acide dobésilique avec une amine de formule (II), dans laquelle $R^1$ et $R^2$ sont égaux et sont des radicaux éthyle.

6. L'acide dobésilique et/ou un sel pharmaceutiquement acceptable, ou un ester de l'un quelconque de l'acide ou du sel, pour l'utilisation selon l'une quelconque des revendications 1 à 5, dans lequel la maladie du segment postérieur

de l'œil est choisie parmi des pathologies de la rétine et/ou de la choroïde, des pathologies de l'humeur vitrée, une pathologie de la sclérotique postérieure, des pathologies du nerf optique, et des combinaisons de celles-ci.

7. L'acide dobésilique et/ou un sel pharmaceutiquement acceptable, ou un ester de l'un quelconque de l'acide ou du sel, pour l'utilisation selon l'une quelconque des revendications 1 à 6, dans lequel la maladie du segment postérieur de l'œil est une pathologie de la rétine et/ou de la choroïde choisie parmi une vasculopathie rétinienne, une maculopathie, une dystrophie héréditaire du fond de l'œil, une choriorétinopathie idiopathique, une rétinopathie séreuse centrale, une dystrophie choroïdienne généralisée, et des combinaisons de celles-ci.

8. L'acide dobésilique et/ou un sel pharmaceutiquement acceptable, ou un ester de l'un quelconque de l'acide ou du sel de celui-ci, pour l'utilisation selon la revendication 7, dans lequel la vasculopathie rétinienne est choisie parmi la rétinopathie diabétique, la papillopathie diabétique, la rétinopathie non diabétique, le syndrome ischémique oculaire, la rétinopathie hypertensive, la rétinopathie thalassémique, le syndrome de Coats, le syndrome de Eales, la rétinopathie radique, la rétinopathie solaire, la rétinopathie de Purtscher, la vasculopathie polypoïdale choroïdienne (VPC), le macroanévrisme rétinien, le microanévrisme rétinien, la rétinopathie leucémique, l'ischémie rétinienne, des troubles chroniques de la rétine, et des combinaisons de ceux-ci.

9. L'acide dobésilique et/ou un sel pharmaceutiquement acceptable, ou un ester de l'un quelconque de l'acide ou du sel, pour l'utilisation selon la revendication 7, dans lequel la maculopathie est choisie parmi la dégénérescence maculaire liée à l'âge (DMLA), la DMLA hémorragique, la prolifération angiomateuse rétinienne, la vasculopathie choroïdienne polipoïdale, la malattia leventinese, un trou maculaire pleine épaisseur, la membrane épirétinienne maculaire, les télangiectasies maculaires, la maculopathie cellophane ou membrane épirétinienne, la maculopathie myopique, la maculopathie exsudative après thrombose veineuse de la rétine, la neurorétinopathie maculaire aiguë, les cystoïdes maculaires, l'"œdème maculaire, les stries angioïdes de la rétine, les plis choroïdiens, la maculopathie d'hypotonie et des combinaisons de ceux-ci; et dans lequel la dystrophie du fond de l'œil héréditaire est choisie dans le groupe constitué de la rétinite pigmentaire; la rétinite pigmentaire atypique comprenant, mais sans s'y limiter, le syndrome d'Usher, la rétinite ponctuée albicans, l'amaurose congénitale de Leber, la dystrophie des cônes, la dystrophie des bâtonnets, la dystrophie cornéorétinienne cristalline de Bietti, la dystrophie maculaire juvénile, tous les types de dystrophie maculaire, la maladie de Stargardt ou Fundus flavimaculatus, le syndrome d'Usher et des combinaisons de ceux-ci.

10. L'acide dobésilique et/ou un sel pharmaceutiquement acceptable, ou un ester de l'un quelconque de l'acide ou du sel, pour l'utilisation selon l'une quelconque des revendications 1 à 6, dans lequel la maladie du segment oculaire postérieur est une pathologie de l'humeur vitrée choisie parmi le groupe comprenant les hémorragies sous-maculaires et vitréennes, l'hyalose astéroïde, le décollement du vitré, les corps flottants du vitré ou myodésopsie, les vitréorétinopathies héréditaires, le syndrome de Stickler ou le syndrome de Wagner et des combinaisons de ceux-ci.

11. L'acide dobésilique et/ou un sel pharmaceutiquement acceptable, ou un ester de l'un quelconque de l'acide ou du sel, pour l'utilisation selon l'une quelconque des revendications 1 à 6, dans lequel la maladie du segment postérieur de l'œil est une pathologie du nerf optique choisie dans le groupe comprenant l'atrophie optique, la névrite optique, la neurorétinite, la neuropathie ischémique, la neuropathie optique héréditaire, l'amblyopie toxique ou la neuropathie optique nutritionnelle, l'hypertension oculaire, le glaucome primitif, le glaucome secondaire, le syndrome endothélial iridocornéen associé au glaucome, le drusen du disque optique ou de la tête du nerf optique, l'œdème papillaire et des combinaisons de ceux-ci.

12. Une composition pharmaceutique topique oculaire pour l'utilisation dans le traitement et/ou la prévention d'une maladie du segment postérieur de l'œil, qui comprend une quantité thérapeutiquement efficace d'acide dobésilique et/ou d'un sel pharmaceutiquement acceptable, ou d'un ester de l'un quelconque de l'acide ou du sel, conjointement avec un ou plusieurs excipients et/ou véhicules topiques pharmaceutiquement acceptables ; dans lequel le traitement comprend l'administration d'une dose topique de l'acide dobésilique et/ou d'un sel pharmaceutiquement acceptable, ou d'un ester de ceux-ci, de 0,01 mg/jour à 400 mg/jour.

13. La composition pharmaceutique topique oculaire pour l'utilisation selon la revendication 12, comprenant de l'acide dobésilique et/ou un sel pharmaceutiquement acceptable, ou un ester de l'un quelconque de l'acide ou du sel, en un pourcentage en poids/volume allant de 0,02 % à 20 % en poids/volume.

14. La composition pharmaceutique topique oculaire pour l'utilisation selon l'une quelconque des revendications 12 à 13, dans laquelle le sel pharmaceutiquement acceptable est choisi parmi un sel de métal alcalin de l'acide dobésilique,

un sel de métal alcalino-terreux de l'acide dobésilique, un sel de l'acide dobésilique avec une amine de formule (II), et des combinaisons de ceux-ci :

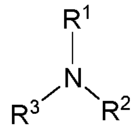 (II),

dans laquelle $R^1$, $R^2$ et $R^3$ sont indépendamment choisis parmi des radicaux alkyle en - ($C_1$-$C_8$)- ou H.

**15.** La composition pharmaceutique topique oculaire pour l'utilisation selon l'une quelconque des revendications 12 à 14, dans laquelle le sel pharmaceutiquement acceptable est le sel de métal alcalino-terreux de l'acide dobésilique dobésilate de calcium.

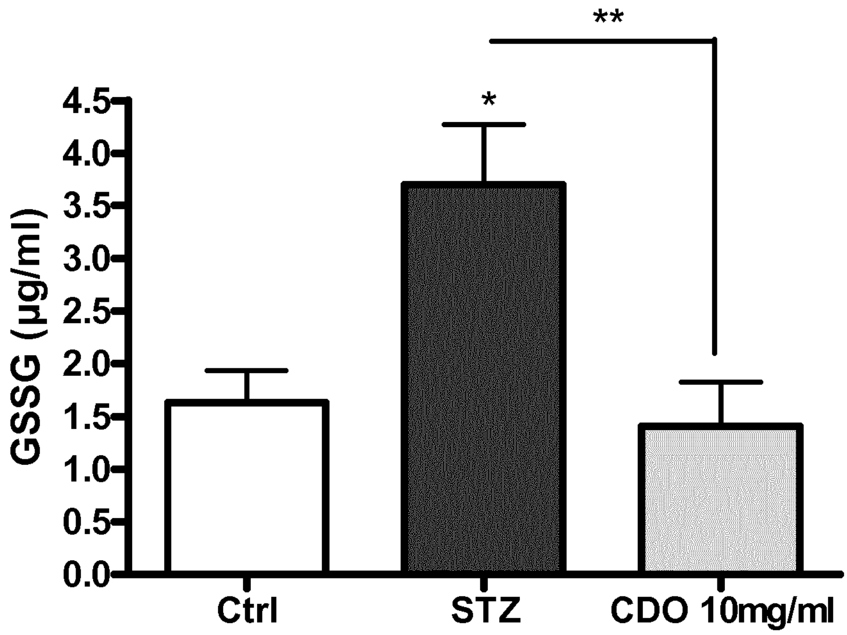


Figure 1

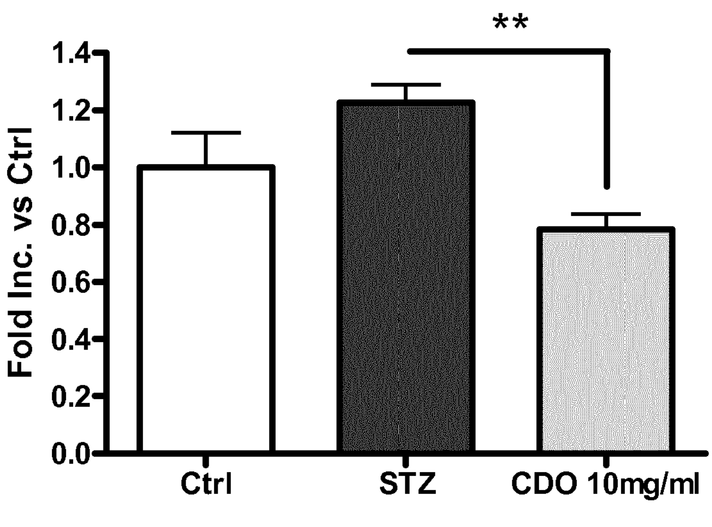


Figure 2

NaCl 0.9% | NaOH 0.1N | CDO (100 mg/ml) | Etamsylate (100 mg/ml) | Potassium dobesilate (100 mg/ml)

T=0

T= 5 min

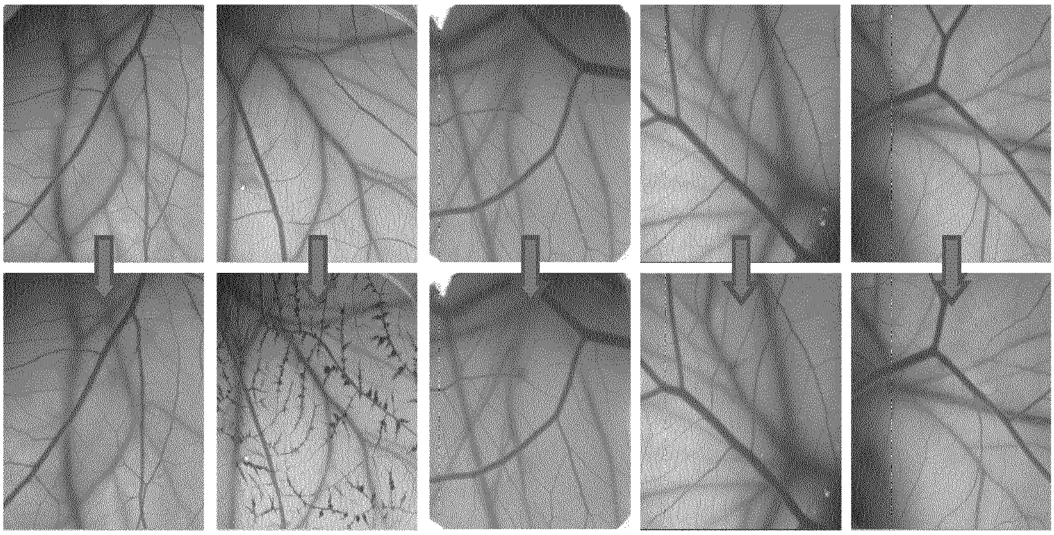

IS | Non irritant | Severe irritant | Non irritant | Non irritant | Non irritant

Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 17382832
- EP 2875811 A1
- EP 2777699 B1
- EP 2875811 A
- EP 2777699 A


### Non-patent literature cited in the description


- **AWWAD et al.** Principles of Pharmacology in the Eye. *British Journal of Pharmacology,* 2017, (174), 4205-4223
- **GAUDANA et al.** Ocular drug delivery. *American Association of Pharmaceutical Sci.,* September 2010, vol. 12 (3), 348-360
- **VADLAPUDI et al.** *Ocular Drug Delivery,* 2019-263
- **CUEVAS et al.** Single Intravitreal Injection of Etamsylate for the Treatment of Geographic Atrophy Associated with Submacular Hemorrhage. *MOJ Clin Med Case,* 2017, vol. 7 (1), 00186
- **CUEVAS et al.** Intravitreal Dobesilate Injection for Macular Oedema Secondary to Branch Retinal Vein Occlusion. *MOJ Clin Med Case,* 2016, vol. 4 (1), 00078
- **SIMÓ et al.** Mechanisms of retinal neuroprotection of calcium dobesilate: therapeutic implications. *Neural Regen Res,* 2017, vol. 12 (10), 1620-1622
- **DEL AMO et al.** Current and future ophthalmic drug delivery systems. A shift to the posterior segment. *Drug Discovery Today,* 2008, vol. 13 (3,4), 135-143
- **RANTA et al.** Barrier analysis of periocular drug delivery to the posterior segment. *Journal of Controlled Release,* 2010, (148), 42-48
- Guidance for Industry Estimating the Maximum Safe Starting Dose in Initial Clinical Trials for Therapeutics in Adult Healthy Volunteers U.S. Department of Health and Human Services Food and Drug Administration Center for Drug Evaluation and Research (CDER). *Pharmacology and Toxicology,* July 2005
- *ICCVAM-Recommended Test Method Protocol,* 2010
- **AWWAD et al.** Principles of Pharmacology in the Eye. *British Journal of Pharmacology,* 2017, vol. 174, 4205-4223
- **VADLAPUDI et al.** *Ocular Drug Delivery,* vol. 10, 2019-263
- **DEL AMO et al.** Current and future ophthalmic drug delivery systems. A shift to the posterior segment. *Drug Discovery Today,* 2008, vol. 13 (3/4), 135-143
- **RANTA et al.** Barrier analysis of periocular drug delivery to theposterior segment. *Journal of Controlled Release,* 2010, (148), 42-48